# EUROPEAN PATENT APPLICATION

(11) **EP 3 187 107 A1**
(43) Date of publication of application: **05.07.2017**
(21) Application number: 15835978.6
(22) Date of filing: 24.08.2015
(51) Int. Cl.: A61B 5/0245

(54) **BIOLOGICAL INFORMATION DETECTION DEVICE**

(30) Priority: 27.08.2014 JP 2014172815
(71) Applicant: Seiko Epson Corporation, Tokyo 160-881 (JP)
(72) Inventor: TANAKA, Shigemitsu, Suwa-shi Nagano 392-8502 (JP); HIDAI, Yoshihiro, Suwa-shi Nagano 392-8502 (JP)
(74) Representative: Hoffmann Eitle
(86) International application number: PCT/JP2015/004226
(87) International publication number: WO 2016/031221

(57) **Abstract**

A biological information detecting device or the like in which waterproof performance or the like is improved by a simple structure is provided.

A biological information detecting device includes a sensor unit 130 which detects biological information of a test body, and a case portion 300 in which the sensor unit 130 is disposed. The case portion 300 is provided with a light transmitting portion 30 including a detection window 120 which transmits light incident on the sensor unit 130, and a light blocking portion 20 blocking light from being incident on an inner portion of the case portion 300. In a case where a direction towards the sensor unit 130 from the test body at the time of mounting the biological information detecting device is set to a first direction DR1, a joining portion 52 between the light transmitting portion 30 and the light blocking portion 20 protrudes or is dented along a direction intersecting with the first direction DR1.

## Description

### Technical Field

The present invention relates to a biological information detecting device or the like.

### Background Art

From the related art, a biological information detecting device detecting biological information such as a human pulse wave has been known. In PTL 1 and PTL 2, a pulse wave measuring device of the related art which is an example of such a biological information detecting device is disclosed.

The device disclosed in PTL 1 and PTL 2 is a photoelectric pulse wave measuring device, and a sensor unit thereof is provided with a light emitting unit emitting light towards a test body which is an object, and a light receiving unit receiving light from the test body (light including biological information). Then, a change in a blood flow rate is detected as a change in the amount of received light by using the sensor unit, and thus, a pulse wave is detected.

### Citation List

### Patent Literature

PTL 1: JP-A-2012-65746
PTL 2: JP-A-2012-139725

### Summary of Invention

### Technical Problem

In such a pulse wave measuring device, it is desirable that waterproof performance is also able to be improved while increasing detection performance of the biological information. For example, in a wrist-mountable pulse wave measuring device, even in a case where there is a body motion such as hand movements at the time of mounting the wrist-mountable pulse wave measuring device, it is necessary that biological information such as a pulse wave is detected with high detection performance. In addition, it is necessary that waterproof performance increases such that sweat, moisture, or the like does not enter the inner portion of the device at the time of mounting the wrist-mountable pulse wave measuring device.

From this viewpoint, in the pulse wave measuring device of PTL 1, a structure is adopted in which a sensor unit of biological information is retained by a retaining unit formed of synthetic rubber or the like. Specifically, the retaining unit retains the sensor unit such that at least a part of the sensor unit of the biological information protrudes from a device main body to the outside. Then, when the pulse wave measuring device is used by being mounted on a wrist of a user, the sensor unit imparts a suitable pressing force to the wrist of the user due to elastic modification of the retaining unit, and thus, detection performance of the biological information is improved. In addition, the main body, a pulse wave sensor unit, and the retaining unit of the pulse wave measuring device have waterproof properties, and thus, the waterproof performance increases.

However, in the related art, a method is adopted in which the sensor unit is retained by the retaining unit formed of synthetic rubber or the like, and thus, the structure of the device becomes complicated. For this reason, the number of components increases, a cost increase in the device is caused, or thinning of the device is difficult to be realized.

According to some aspects of the present invention, it is possible to provide a biological information detecting device or the like in which waterproof performance or the like is improved by a simple structure.

### Solution to Problem

An aspect of the present invention relates to a biological information detecting device, including: a sensor unit which detects biological information of a test body; and a case portion in which the sensor unit is disposed, in which the case portion is provided with a light transmitting portion including a detection window which transmits light incident on the sensor unit, and a light blocking portion blocking light from being incident on an inner portion of the case portion, and in a case in which a direction towards the sensor unit from the test body is set to a first direction at the time of mounting the biological information detecting device, a joining portion between the light transmitting portion and the light blocking portion protrudes or is dented along a direction intersecting with the first direction.

In the aspect of the present invention, the sensor unit detecting the biological information of the test body is disposed in the case portion, and the case portion is provided with the light transmitting portion including the detection window which transmits the light incident on the sensor unit, and the light blocking portion blocking the light from being incident on the inner portion of the case portion. Then, the joining portion between the light transmitting portion and the light blocking portion protrudes or is dented along the direction intersecting with the first direction towards the sensor unit from the test body. Accordingly, in the joining portion between the light transmitting portion for the sensor unit and the light blocking portion for blocking unnecessary light, it is possible to improve fixing performance, to increase a joining area, and the like. Accordingly, waterproof performance or the like of the biological information detecting device is improved while joining the light transmitting portion to the light blocking portion by a simple structure.

In addition, in the aspect of the present invention, the light transmitting portion may include a first convex portion protruding in a direction opposite to the first direction.

In addition, in the aspect of the present invention, a second convex portion disposed to surround the light transmitting portion may be provided.

In addition, in the aspect of the present invention, the light transmitting portion may include a first convex portion which imparts a pressing force by being in contact with the test body at the time of measuring the biological information, and the light blocking portion may include a second convex portion which suppresses the pressing force imparted to the test body by the first convex portion.

Accordingly, the pressing force imparted to the test body by the first convex portion of the light transmitting portion is suppressed by the second convex portion, and thus, it is possible to reduce a variation in the pressing force, to improve detection performance of the biological information, and the like.

In addition, in the aspect of the present invention, the joining portion may protrude or may be dented along the direction intersecting with the first direction on the first direction side of the second convex portion.

Accordingly, even in a case where an external force caused by suppressing the pressing force is imparted to the second convex portion, the joining portion between the light transmitting portion and the light blocking portion protrudes or is dented in the direction intersecting with the first direction on the first direction side of the second convex portion, and thus, it is possible to effectively suppress an adverse effect of the external force.

In addition, in the aspect of the present invention, in a case in which a direction opposite to the first direction is set to a second direction, and a value obtained by subtracting a height of the second convex portion in the second direction from a height of the first convex portion in the second direction is set to Δh, Δh > 0 may be satisfied.

Thus, the first convex portion of the light transmitting portion protrudes to be Δh, and thus, it is possible to impart a suitable initial pressing force to the test body by the first convex portion with a small load. Then, the pressing force imparted by the first convex portion is suppressed by the second convex portion, it is possible to reduce a variation in the pressing force, and the like. Accordingly, it is possible to provide a biological information detecting device in which a suitable initial pressing force is able to be imparted to the test body while reducing an adverse effect of a variation in the pressing force, and the like.

In addition, in the aspect of the present invention, in a case in which a change amount of the pressing force of the first convex portion with respect to a load of a load mechanism generating the pressing force of the first convex portion is set to a pressing force change amount, the second convex portion may suppress the pressing force imparted to the test body by the first convex portion such that the pressing force change amount in a second load range in which the load of the load mechanism becomes greater than FL1 may become smaller than the pressing force change amount in a first load range in which the load of the load mechanism becomes 0 to FL1.

Accordingly, it is possible to reduce a variation in the pressing force by suppressing the pressing force imparted to the test body by the first convex portion by the second convex portion while imparting a suitable initial pressing force to the test body by the first convex portion.

In addition, in the aspect of the present invention, a groove portion may be disposed between the first convex portion of the light transmitting portion and the second convex portion.

In a case where such a groove portion is disposed, it is possible to suppress a dynamic change or the like in a contact state around the first convex portion, to concentrate a load on the first convex portion at the time of imparting an initial pressing force, and the like. Accordingly, it is possible to effectively suppress deterioration or the like in signal integrity of a detection signal of the light receiving unit due to a change in the contact state or the like.

In addition, in the aspect of the present invention, the light transmitting portion and the light blocking portion may be integrally formed by two-color molding or insert molding.

Thus, in a case where the light transmitting portion is joined to the light blocking portion by the two-color molding or the insert molding, waterproof performance or the like of the biological information detecting device is improved by a simple joining structure.

In addition, in the aspect of the present invention, the light transmitting portion may be formed of a material including a first resin material, and the light blocking portion may also be formed of the material including the first resin material.

Thus, in a case where the light transmitting portion and the light blocking portion are formed of the same material including the first resin material, for example, it is possible to matching a melting point, and to improve fixing performance or the like between the light transmitting portion and the light blocking portion in the joining portion.

In addition, in the aspect of the present invention, the light blocking portion may be formed of a material in which glass is contained in the first resin material.

Thus, in a case where the light blocking portion is formed of a glass-containing resin material, it is possible to increase strength of the light blocking portion, and thus, it is possible to suppress inflow of moisture due to modification or a damage in an inner component. In addition, it is not necessary to increase a thickness of a member at the time of increasing the strength, and thus, thinning and weight saving of the biological information detecting device are easily realized.

In addition, in the aspect of the present invention, the first resin material may be polycarbonate, an ABS resin, or an acrylic resin.

Accordingly, the light transmitting portion and the light blocking portion are formed of a material including the polycarbonate, the ABS resin, or the acrylic resin, which is the first resin material, and thus, for example, it is possible to match a melting point, and to improve fixing performance or the like between the light transmitting portion and the light blocking portion in the joining portion.

In addition, in the aspect of the present invention, the light blocking portion may be joined to the light transmitting portion by being disposed to surround the detection window of the light transmitting portion, and the joining portion between the light transmitting portion and the light blocking portion may protrude or may be dented in a direction towards the light blocking portion from the detection window of the light transmitting portion.

Accordingly, the joining portion in which the light transmitting portion is joined to the light blocking portion around the detection window has a structure in which the joining portion protrudes or is dented in the direction towards the light blocking portion from the detection window, and thus, waterproof performance or the like in the joining portion around the detection window is improved.

In addition, in the aspect of the present invention, the light transmitting portion may include a convex portion protruding in a direction towards the light blocking portion from the detection window of the light transmitting portion, and the light blocking portion may include a concave portion engaged with the convex portion of the light transmitting portion.

Accordingly, the convex portion of the light transmitting portion protruding in the direction towards the light blocking portion from the detection window is engaged with the concave portion of the light blocking portion, and thus, it is possible to realize the joining portion protruding in the direction towards the light blocking portion from the detection window.

In addition, in the aspect of the present invention, the light blocking portion may include a convex portion protruding in a direction towards the detection window of the light transmitting portion from the light blocking portion, and the light transmitting portion may include a concave portion engaged with the convex portion of the light blocking portion.

Accordingly, the convex portion of the light blocking portion protruding in the direction towards the detection window from the light blocking portion is engaged with the concave portion of the light transmitting portion, and thus, it is possible to realize the joining portion dented in the direction towards the detection window from the light blocking portion.

In addition, in the aspect of the present invention, the case portion may be provided with a third convex portion which prevents at least one of the light transmitting portion and the light blocking portion from being modified in the first direction in the joining portion.

Accordingly, for example, the light transmitting portion or the light blocking portion is prevented from being modified in the first direction by the third convex portion, and thus, it is possible to maintain fixing performance in the joining portion between the light transmitting portion and the light blocking portion, and the like.

In addition, in the aspect of the present invention, a third convex portion may be disposed between a circuit case supporting a circuit substrate and the light transmitting portion.

In addition, in the aspect of the present invention, the sensor unit may include a light emitting unit emitting light to the test body, and a light receiving unit receiving light from the test body.

Accordingly, the biological information is able to be detected by using the photoelectric sensor.

In addition, in the aspect of the present invention, the biological information may be pulse wave information.

Here, the biological information which becomes a detection target of the biological information detecting device is not limited to the pulse wave.

### Brief Description of Drawings

[Fig. 1] Fig. 1(A) and Fig. 1(B) are perspective views illustrating an example of a whole configuration of a biological information detecting device.
[Fig. 2] Fig. 2(A) and Fig. 2(B) are plan views illustrating the example of the whole configuration of the biological information detecting device.
[Fig. 3] Fig. 3 is a development view schematically illustrating a structure of the biological information detecting device.
[Fig. 4] Fig. 4 is a diagram schematically illustrating a configuration example of the biological information detecting device in which a method of this embodiment is realized.
[Fig. 5] Fig. 5 is a diagram schematically illustrating a second configuration example of the biological information detecting device in which the method of this embodiment is realized.
[Fig. 6] Fig. 6 is an explanatory diagram illustrating improvement in strength realized by containing glass.
[Fig. 7] Fig. 7 is an example of a configuration of a biological information detecting device of a comparative example.
[Fig. 8] Fig. 8 is a sectional view illustrating a specific example of the biological information detecting device.
[Fig. 9] Fig. 9 (A) to Fig. 9(D) are plan views, a sectional view, and a side view illustrating a specific example of a rear cover portion.
[Fig. 10] Fig. 10 is a sectional view illustrating a specific example in the vicinity of a sensor unit of the biological information detecting device.
[Fig. 11] Fig. 11(A) and Fig. 11(B) are explanatory diagrams of a problematic point when a pressing force of a light transmitting portion with respect to a test body is changed.
[Fig. 12] Fig. 12 is an explanatory diagram illustrating pressing force suppression of a pressing force suppressing unit.
[Fig. 13] Fig. 13 is a sectional view illustrating one example of the biological information detecting device including a modification suppressing unit.
[Fig. 14] Fig. 14 is a sectional view illustrating another example of the biological information detecting device including the modification suppressing unit.
[Fig. 15] Fig. 15 is a functional block diagram illustrating a configuration example of the biological information detecting device.
[Fig. 16] Fig. 16 is a functional block diagram illustrating another configuration example of the biological information detecting device.

### Description of Embodiments

Hereinafter, this embodiment will be described. Furthermore, this embodiment described below does not unjustly limit the contents of the present invention disclosed in Claims. In addition, the whole configuration described in this embodiment is not the essential requirements of the present invention.

### 1. Whole Configuration of Biological Information Detecting Device

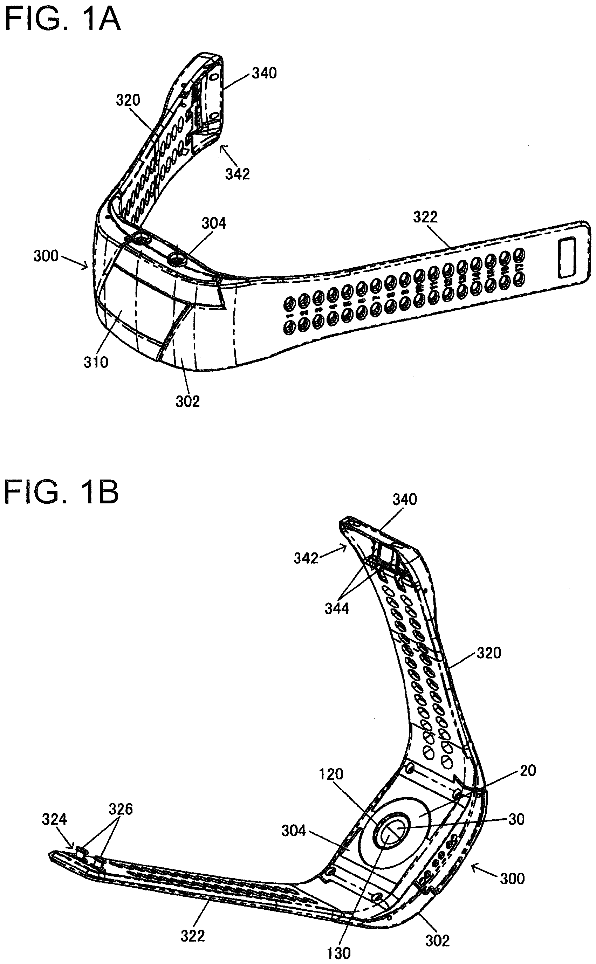

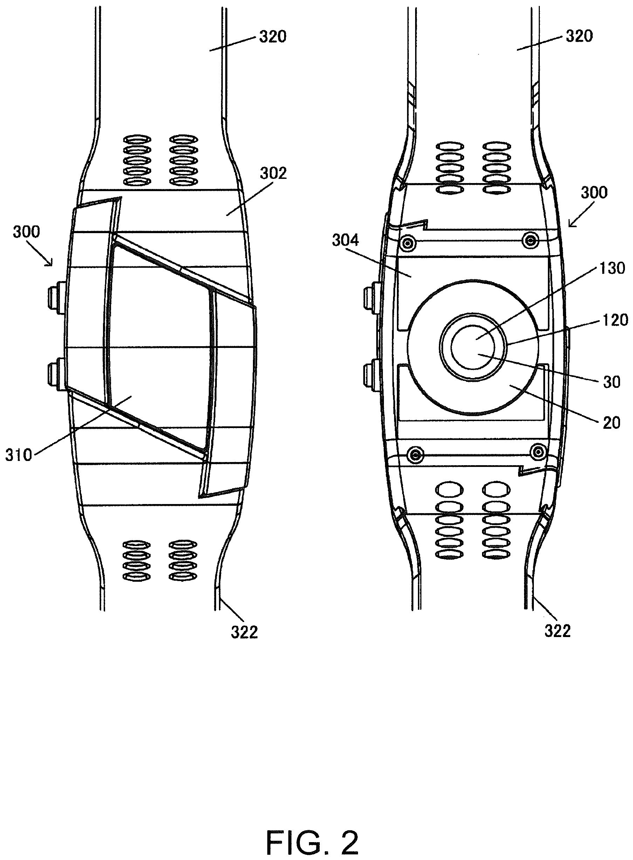

First, an example of a whole configuration of a biological information detecting device of this embodiment will be described. Fig. 1(A) and Fig. 1(B) are perspective views illustrating one example of the biological information detecting device, and Fig. 2(A) and Fig. 2(B) are plan views illustrating one example of the biological information detecting device. Fig. 1(A) and Fig. 2(A) are a perspective view and a plan view of the biological information detecting device seen from a front side (a display unit side), respectively, and Fig. 1(B) and Fig. 2(B) are a perspective view and a plan view of the biological information detecting device seen from a rear side, respectively. The biological information detecting device (a biological information measuring device) is a wrist-mountable (watch-type) device, is mounted on a wrist of a user (in a broad sense, a given portion), and detects biological information such as pulse wave information. The biological information detecting device includes a case portion 300 (a main body portion), and band portions 320 and 322 for mounting the case portion 300 on the user.

Furthermore, hereinafter, a case where the biological information detecting device is a wrist-mountable pulse monitor will be described as an example, but this embodiment is not limited thereto. For example, the biological information detecting device of this embodiment may be mounted on a portion other than the wrist (for example, a leg, an upper arm, a finger, a chest, and the like) and may detect (measure) the biological information. In addition, the biological information which becomes a detection target of the biological information detecting device is not also limited to a pulse wave (a pulse rate), and the biological information detecting device may be a device detecting biological information other than the pulse wave (for example, an oxygen saturation in the blood, a body temperature, a heart rate, a blood pressure, and the like).

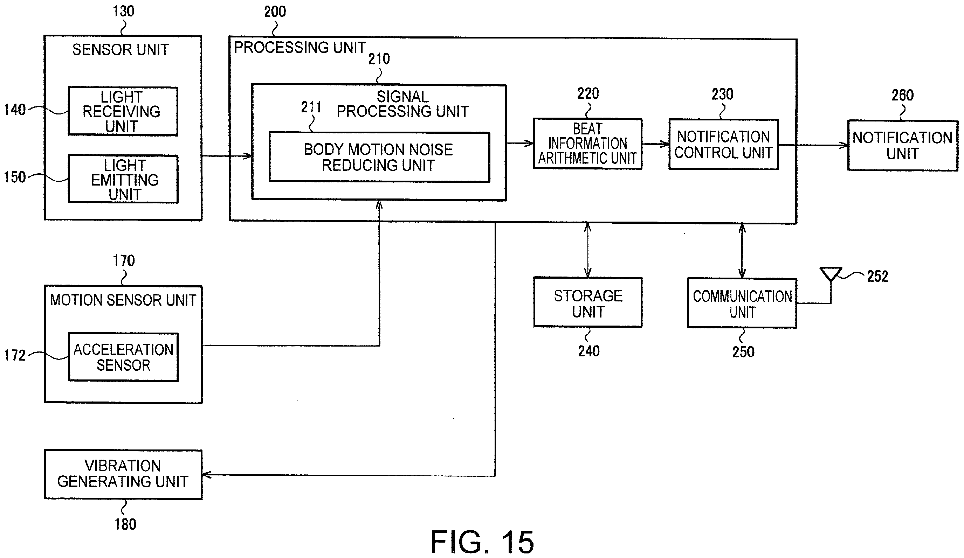

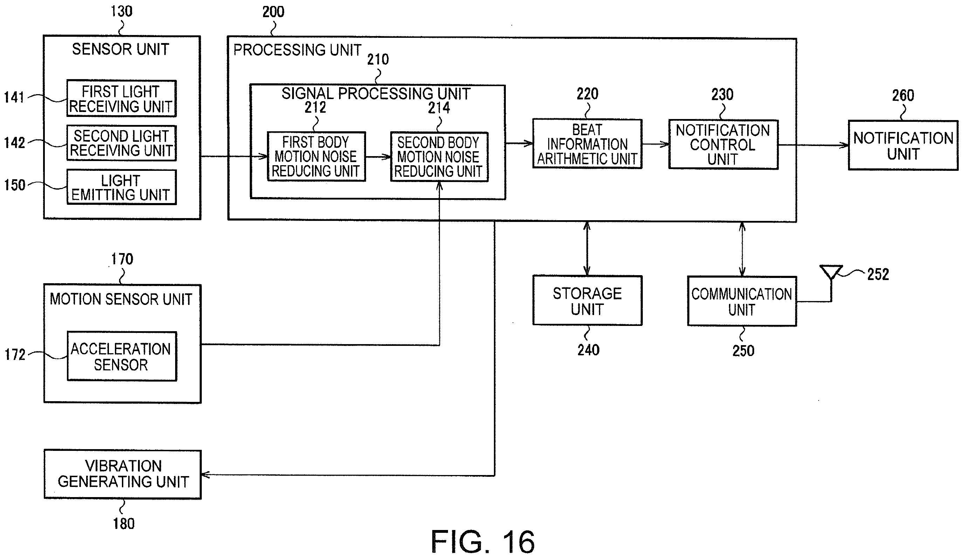

The case portion 300 contains each unit of the biological information detecting device such as a sensor unit 130 or a processing unit 200 described below in Fig. 15 and Fig. 16. The case portion 300 is formed of a main body portion which adheres to the user and detects the biological information. In Fig. 1(A) to Fig. 2(B), the case portion 300 (the main body portion) is configured of a top case 302 and a rear cover portion 304 (a bottom case). A display unit 310 such as LCD is disposed in the top case 302. Various information items such as a pulse rate (in a broad sense, biological detection information), calory consumption (in a broad sense, active state information), or a time are displayed on the display unit 310.

Furthermore, the case portion 300 may not have such a structure in which the case portion 300 is divided into the top case 302 and the rear cover portion 304. For example, the case portion 300 may be formed of an integrated member. In addition, the biological information detecting device, for example, may be subjected to communication connection with respect to an external terminal device (not illustrated), and may enable data exchange. In this case, the terminal device, for example, is a portable communication terminal such as a smart phone, a mobile phone, and a feature phone. Alternatively, the terminal device may be an information processing terminal such as a tablet type computer. In the communication connection between the biological information detecting device and the terminal device, for example, proximity wireless communication such as Bluetooth (Registered Trademark) is able to be adopted. Thus, the communication connection between the biological information detecting device and the terminal device is performed, and thus, various information items such as a pulse rate or calory consumption are able to be displayed on a display unit (LCD or the like) of the terminal device. Furthermore, arithmetic processing of the information such as a pulse rate or calory consumption may be executed by the biological information detecting device, or at least a part thereof may be executed in the terminal device.

A plurality of hole portions are formed in the band portions 320 and 322. The band portion 320 includes a buckle portion 340. The buckle portion 340 includes a band insertion portion 342 and a rod portion 344. In a case where the biological information detecting device is mounted on the wrist of the user, the band portion 322 is inserted into the band insertion portion 342 of the buckle portion 340. Then, the rod portion 344 (two rod portions) of the buckle portion 340 is inserted into the hole portion (two hole portions) of the band portion 322, and is fixed thereto. In addition, a pin 326 of a hook portion 324 of the band portion 322 is inserted into the hole portion of the band portion 320, and the band portion 322 is locked in the band portion 320. Accordingly, the biological information detecting device is mounted on the wrist of the user.

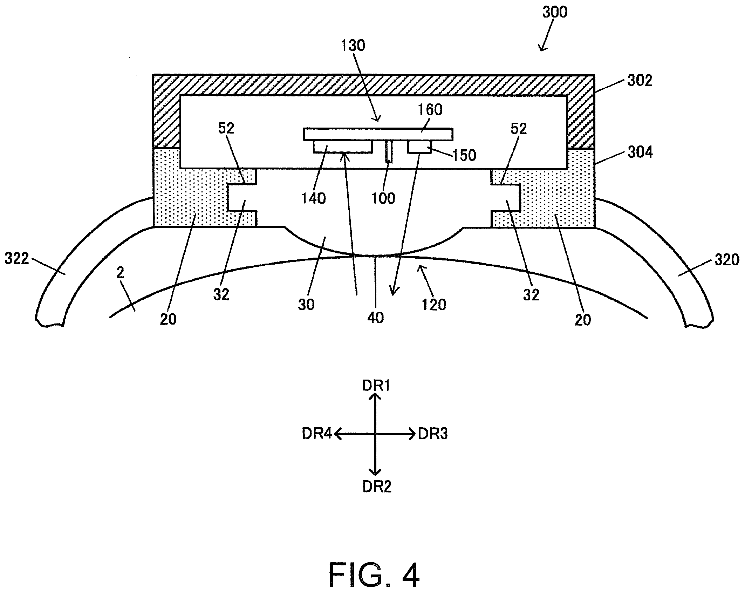

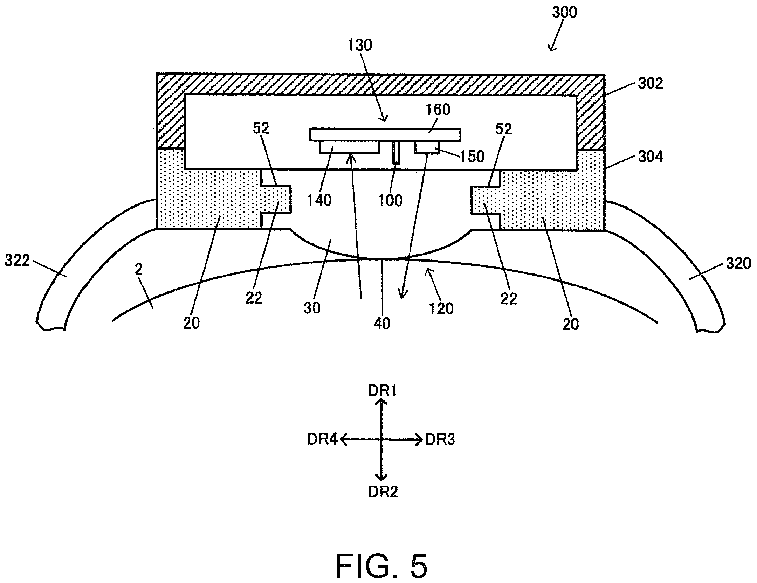

The band portions 320 and 322, for example, are formed of an elastic material including a polyurethane resin or a silicone resin, and have stretching properties (and flexibility) for adhesively mounting the case portion 300 (the device main body) on the wrist or the like of the user. Then, a load mechanism of this embodiment is realized by fastening the band portions 320 and 322 which are elastic members, and the like. That is, an elastic force of the band portions 320 and 322 acts, and thus, it is possible to ensure to some extent that a pressing force imparted to a test body by a convex portion 40 of a light transmitting portion 30 described below in Fig. 4, Fig. 5, and the like becomes an assumed suitable pressing force.

Next, the sensor unit 130 of this embodiment will be described. The biological information detecting device of this embodiment includes the sensor unit 130 which detects the biological information (the pulse wave and the like) of the test body, and the case portion 300 in which the sensor unit 130 is disposed. As illustrated in Fig. 4 and the like described below, the sensor unit 130, for example, is able to include a light emitting unit 150 emitting light to the test body, and a light receiving unit 140 receiving light from the test body, and the like. The light emitting unit 150 and the light receiving unit 140 are mounted on a substrate 160, and are contained in the inner portion of the case portion 300.

Then, as illustrated in Fig. 1(B) and Fig. 2(B), the light transmitting portion 30 and a light blocking portion 20 are disposed in the case portion 300. For example, the light transmitting portion 30 and the light blocking portion 20 are disposed on the surface side of the case portion 300 on the test body side. Here, the surface of the case portion 300 on the test body side is a housing surface facing the test body (the wrist or the like) at the time of mounting the biological information detecting device. Specifically, the rear cover portion 304 including the surface of the case portion 300 on the test body side is configured of the light transmitting portion 30 and the light blocking portion 20.

The light transmitting portion 30 includes a detection window 120 which transmits light incident on the sensor unit 130. The light transmitting portion 30 transmits light from the test body, and imparts a pressing force by being in contact with the test body at the time of measuring the biological information of the test body. For example, the light transmitting portion 30 transmits light incident on the light receiving unit 140 (the light from the test body) in Fig. 4 described below. In addition, the light transmitting portion 30 transmits light emitted from the light emitting unit 150. Then, in a case where a direction towards the sensor unit 130 from the test body is set to a first direction at the time of mounting the biological information detecting device, the light transmitting portion 30 includes a convex portion 40 (in other words, a first convex portion, and the same hereinafter) protruding in a direction opposite to the first direction. For example, the light transmitting portion 30 includes the convex portion 40 (the first convex portion) imparting a pressing force by being in contact with the test body at the time of measuring the biological information.

The light blocking portion 20 (a cover member) blocks light from being incident on the inner portion of the case portion 300. For example, unnecessary light is blocked from being incident on the sensor unit 130. As illustrated in Fig. 1(B) and Fig. 2(B), the light blocking portion 20 is disposed to surround the detection window 120 of the light transmitting portion 30. For example, a part of the light transmitting portion 30 is exposed to the test body side from an opening of the light blocking portion 20, and in this exposed portion, the convex portion 40 in Fig. 4 is formed. Accordingly, the convex portion 40 formed in the exposed portion is brought into contact with the test body (for example, the skin of the wrist of the user) at the time of measuring the biological information. The detection window 120 is configured by the exposed portion of the light transmitting portion 30. Then, the sensor unit 130 is disposed in a position corresponding to the detection window 120.

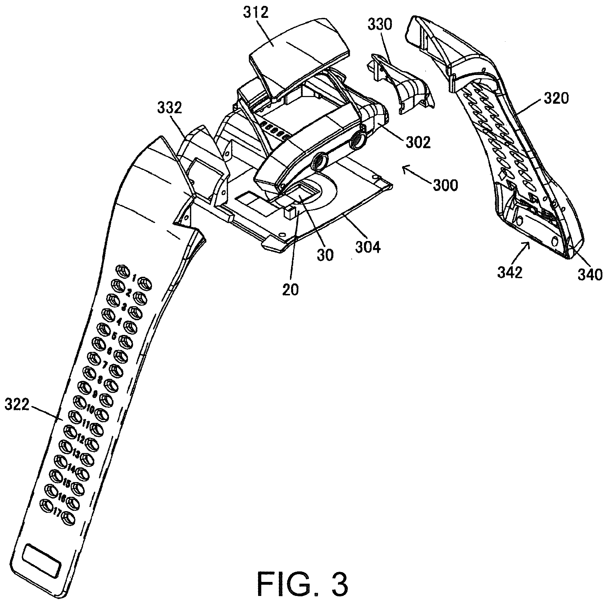

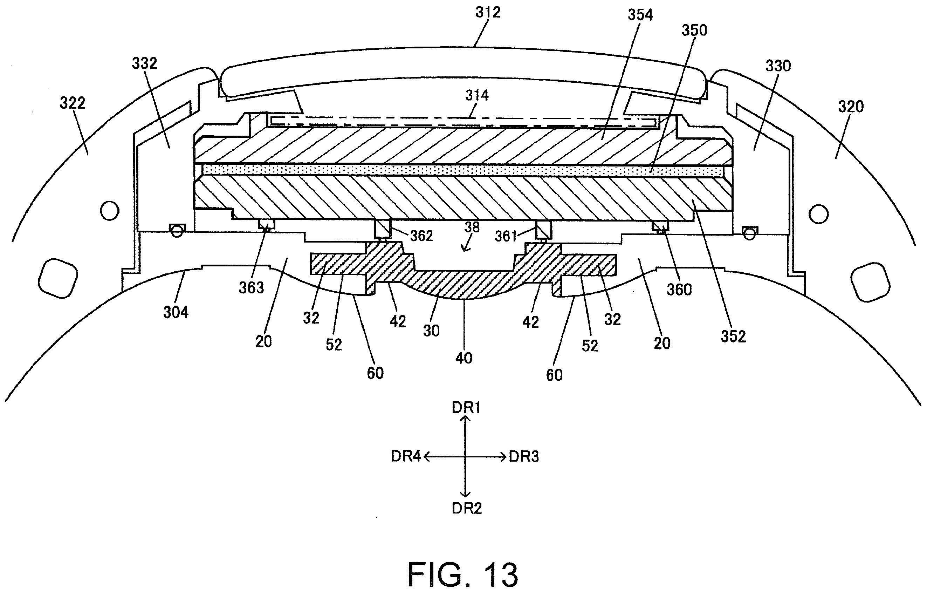

Fig. 3 is a development view schematically illustrating the structure of the biological information detecting device. A module (not illustrated) is incorporated in the inner portion of the case portion 300 configured of the top case 302 and the rear cover portion 304 (the bottom case). The module includes a circuit substrate 350, a circuit case 352, a panel frame 354, and a display panel 314 in Fig. 13 described below, or the sensor unit 130. A windshield 312 of the display unit 310 is attached to the top case 302. The rear cover portion 304 is configured of the light transmitting portion 30 and the light blocking portion 20. Attachment members 330 and 332 are members for attaching the band portions 320 and 322 to the case portion 300 (the device main body). For example, a spring rod (not illustrated) is inserted into a hole portion disposed in a lug of the case portion 300 on the band portion 320 side, a hole portion disposed in the attachment member 330, and a hole portion disposed in an end portion of the band portion 320, and thus, the band portion 320 is attached to the case portion 300. In addition, a spring rod (not illustrated) is inserted into a hole portion disposed in a lug of the case portion 300 on the band portion 322 side, a hole portion disposed in the attachment member 332, and a hole portion disposed in an end portion of the band portion 322, and thus, the band portion 322 is attached to the case portion 300.

### 2. Method of This Embodiment

Next, a method of this embodiment will be described. Fig. 4 is a sectional view schematically illustrating the configuration of the biological information detecting device in which the method of this embodiment is realized.

In a wearable biological information detecting device to be mounted on the wrist or the like of the user, a method of acquiring biological information by using a photoelectric sensor is known. For example, a pulse wave sensor is considered as a biological sensor which is a photoelectric sensor, and pulse wave information such as a pulse rate is able to be acquired by using the pulse wave sensor. Furthermore, the sensor unit 130 which is a biological sensor is not limited to the pulse wave sensor, a photoelectric sensor or the like acquiring biological information other than the pulse wave information may be used.

In the biological information detecting device including the photoelectric sensor, it is necessary to receive necessary light, and to block unnecessary light. According to an example of the pulse wave sensor, reflected light reflected on the test body (in particular, a portion including a blood vessel of a measurement target) includes a pulse wave component, and thus, is required to be received, but the other light becomes a noise component, and thus, is required to be blocked. Here, direct light which is emitted from the light emitting unit 150 and is directly incident on the light receiving unit 140, reflected light reflected on a portion other than the test body, or environment light such as sunlight or illumination light is considered as "the other light".

In order to suitably control such light transmission and light blocking, it is desirable that the light transmitting portion 30 and the light blocking portion 20 are disposed. For example, in Fig. 4, for example, an arrangement relationship between the light transmitting portion 30 and the light blocking portion 20 on the surface side of the case portion 300 on the test body side, and the like may be considered.

Here, in a case where a use case of the biological information detecting device according to this embodiment is assumed, a suitable device is able to be realized by satisfying various requirements other than the arrangement relationship between the light transmitting portion 30 and the light blocking portion 20.

First, the biological information detecting device is required to have high waterproof performance. This is because a component (a module) such as a circuit substrate, a battery, or a display panel is incorporated in the inner portion of the biological information detecting device (according to the example of Fig. 4, a space between the top case 302 and the rear cover portion 304), and thus, in a case where waterproof performance is low, a failure may occur in the component. In particular, it is also considered that a wrist-mountable wearable device or the like is mounted at the time of exercising, and is used for information presentation of exercise strength or the like. In this case, there are many cases where the skin surface of the user is in a sweat, and it is desirable to suppress a risk in which a liquid or gas such as moisture vapor flow flows into a device inner portion.

Second, it is necessary that the strength of the case portion 300 (the top case 302 and the rear cover portion 304) is high. As described above, various components are arranged in the device inner portion, and various forces are applied to the wearable biological information detecting device according to the movement of the user. For example, in a case where the user is jogging, or the like, a pushing force or a twisting force is applied to the device according to the movement of swinging the arms. In this case, in a case where the forces are applied to an inner component such as a circuit substrate, a failure occurs in the component.

Third, it is necessary that mounting feeling of the user is excellent. It is necessary that the wearable biological information detecting device is mounted on the user at the time of being used. As described above, in a case where the wearable biological information detecting device is used at the time of exercising, it is necessary that the wearable biological information detecting device is continuously mounted for a period in which data is required to be acquired (for example, from the start to the end of the exercise). Alternatively, according to an example in which a health degree of the user is determined, it is necessary that the device is mounted for a long period of time (for example, a span such as 12 hours, 24 hours, and several days), and the biological information is continuously acquired. For this reason, it is not preferable that the exercise or the daily living of the user is disturbed by mounting the biological information detecting device, and thus, excellent mounting feeling becomes an important factor. Specifically, it is desirable that the biological information detecting device is small (thin), and is light.

That is, it is desirable that the biological information detecting device is able to receive necessary light and to block unnecessary light, has high waterproof properties and high strength, and is small and light. Fig. 4 is a sectional view schematically illustrating the configuration of the biological information detecting device of this embodiment in which such requirements are realized.

As illustrated in Fig. 4, the biological information detecting device of this embodiment includes the sensor unit 130 which detects the biological information of the test body, and the case portion 300 in which the sensor unit 130 is disposed. The sensor unit 130 includes the light emitting unit 150 emitting light to the test body, and the light receiving unit 140 receiving light from the test body. In addition, the sensor unit 130 includes the substrate 160 (a sensor substrate) on which the light emitting unit 150, the light receiving unit 140, a light blocking wall 100 blocking direct light from the light emitting unit 150 from being incident on the light receiving unit 140, and the like are mounted. Furthermore, the light receiving unit 140 and the like are mounted on the substrate 160, and a modification in which the light emitting unit 150 is not mounted on the substrate 160 (for example, the light emitting unit 150 is mounted on another substrate) is also able to be performed. In addition, in Fig. 4, a case is illustrated in which the number of light receiving units or light emitting units is one, and the number of light receiving units or light emitting units may be greater than or equal to two. For example, a plurality of light receiving units may be disposed corresponding to one light emitting unit.

The light emitting unit 150 emits light to the test body (in a broad sense, an object), and the light receiving unit 140 receives light from the test body. For example, in a case where the light emitting unit 150 emits light, and the light is reflected on the test body, the light receiving unit 140 receives the reflected light. The light receiving unit 140, for example, is able to be realized by a receiving element such as a photodiode. The light emitting unit 150, for example, is able to be realized by a light emitting element such as LED. For example, the light receiving unit 140 is able to be realized by a P-N junction diode element formed on a semiconductor substrate. In this case, it is desirable that an angle limiting filter limiting an incidence angle of the light incident on a receiving region of the light receiving unit 140 or a wavelength limiting filter limiting a wavelength of the light incident on the receiving element is formed on the diode element.

In a case where a case of being applied to the biological information detecting device such as a pulse monitor is used as an example, light from the light emitting unit 150 proceeds to the inner portion of the test body, and is diffused or scatters in epidermis, dermis, a hypodermal tissue, and the like. After that, the light reaches a blood vessel (a detected portion) and is reflected. At this time, a part of the light is absorbed by the blood vessel. Then, an absorption rate of light in the blood vessel is changed according to an influence of a pulse, and a light intensity of the reflected light is also changed, and thus, the light receiving unit 140 receives the reflected light, and detects a change in the light intensity, and thus, a pulse rate or the like which is biological information is able to be detected.

The case portion 300 includes the top case 302 and the rear cover portion 304 (the bottom case). The sensor unit 130 is contained in the inner portion of the case portion 300 which is hermetically closed by the top case 302 and the rear cover portion 304. Specifically, in Fig. 3, the sensor unit 130 (the substrate 160) is attached to a position corresponding to the light transmitting portion 30 of the rear cover portion 304.

The light transmitting portion 30 including the detection window 120 which transmits light incident on the sensor unit 130, and the light blocking portion 20 which blocks light from being incident on the inner portion of the case portion 300 are disposed in the case portion 300. For example, the light transmitting portion 30 and the light blocking portion 20 are disposed on the surface side of the case portion 300 on the test body side. Specifically, the rear cover portion 304 of the case portion 300 is configured of the light transmitting portion 30 and the light blocking portion 20 (the cover member).

The light transmitting portion 30 is disposed in a position on the test body side further than the light receiving unit 140 and the light emitting unit 150. For example, the light transmitting portion 30 is disposed on the surface side of the biological information detecting device in contact with the test body (the housing surface side of the rear cover portion). Then, the light transmitting portion 30 transmits light from the test body, and imparts a pressing force by being in contact with the test body at the time of measuring the biological information of the test body. For example, the light transmitting portion 30 transmits light incident on the light receiving unit 140 (light from the test body). In addition, the light transmitting portion 30 transmits light emitted from the light emitting unit 150. Then, the light transmitting portion 30 includes the convex portion 40 (the first convex portion) which imparts a pressing force by being in contact with the test body at the time of measuring the biological information of the test body. Furthermore, it is desirable that the surface shape of the convex portion 40 is a curved surface shape (a spherical surface shape), but the surface shape is not limited thereto, and various shapes are able to be adopted. In addition, the light transmitting portion 30 may be transparent with respect to the wavelength of the light from the test body, and a transparent material may be used, or a colored material may be used.

The light blocking portion 20 is disposed to surround the detection window 120 of the light transmitting portion 30 and is joined to the light transmitting portion 30. For example, an opening for exposing the detection window 120 to the outside is disposed in the light blocking portion 20 which is a cover member, and the detection window 120 of the light transmitting portion 30 is formed in the opening portion. The sensor unit 130 is disposed in a position of the detection window 120, and the light from the test body is incident on the sensor unit 130 through the detection window 120 of the light transmitting portion 30. On the other hand, a portion other than the opening portion in the rear cover portion 304 becomes the light blocking portion 20, and blocks light from being incident on the inner portion of the case portion 300. That is, the light blocking portion 20 blocks light from being incident on the sensor unit 130 from a portion other than the detection window 120. For example, the light blocking portion 20 is formed of a colored (for example, black) resin, and thus, blocks light.

The light transmitting portion 30 is joined to the light blocking portion 20 by the joining portion 52. The joining portion 52 has a shape along the circumference of the detection window 120 in a planar view seen from the test body side (a planar view in a first direction DR1) of Fig. 2(B). Specifically, in the planar view seen from the test body side, the joining portion 52 has a shape along an outer circumference of a circular shape (including an approximately circular shape) of the detection window 120.

Then, in this embodiment, as illustrated in Fig. 4, the joining portion 52 between the light transmitting portion 30 and the light blocking portion 20 protrudes along a direction towards the light blocking portion 20 from the detection window 120 of the light transmitting portion 30. Alternatively, as illustrated in a second configuration example of this embodiment of Fig. 5, the joining portion 52 is dented along the direction towards the light blocking portion 20 from the detection window 120 of the light transmitting portion 30.

For example, in Fig. 4, the first direction DR1 is a direction towards the sensor unit 130 (the biological information detecting device) from the test body (in a broad sense, an object) at the time of mounting the biological information detecting device. In other words, the first direction DR1 is a direction towards the top case 302 from the rear cover portion 304. A second direction DR2 is a direction opposite to the first direction DR1. Alternatively, the second direction DR2 is a direction perpendicular to a receiving surface of the light receiving unit 140 or a direction parallel to an optical axis of the light emitting unit 150. A third direction DR3 is a direction intersecting with (in a narrow sense, orthogonal to) the first direction DR1 (and the second direction DR2). A fourth direction DR4 is a direction opposite to the third direction DR3, and is a direction intersecting with (orthogonal to) the first direction DR1 (and the second direction DR2).

Then, in the configuration example of Fig. 4, the joining portion 52 between the light transmitting portion 30 and the light blocking portion 20 protrudes along the third direction DR3 or the fourth direction DR4 intersecting with the first direction DR1. For example, the joining portion 52 protrudes in a direction towards the light blocking portion 20 from the detection window 120. Specifically, the joining portion 52 protrudes in a direction towards the outer circumference of the detection window 120 from the center of the detection window 120. For example, in the sectional view of Fig. 4, the joining portion 52 protrudes into the sectional shape of a convex shape. For example, in Fig. 4, the joining portion 52 includes a first joining surface and a second joining surface along the directions (DR3 and DR4) intersecting with the first direction DR1, and a third joining surface intersecting with (orthogonal to) the first joining surface and the second joining surface. In the sectional view of Fig. 4, the sectional shape of the first joining surface, the second joining surface, and the third joining surface of the joining portion 52 is a convex shape.

In addition, in the plan view of Fig. 2(B), the light blocking portion 20 is disposed to surround the detection window 120 of the light transmitting portion 30, and is joined to the light transmitting portion 30. In this case, the jointing portion 52 protrudes along the direction towards the light blocking portion 20 from the detection window 120 of the light transmitting portion 30. Specifically, in the detection window 120 having a circular shape, the joining portion 52 protrudes in a direction towards the outer circumference from the center. Furthermore, it is not necessary that the joining portion 52 protrudes in the entire position of the outer circumference of the detection window 120, and a region in which the joining portion 52 does not protrude may be included in the outer circumference.

In addition, in Fig. 5 which is the second configuration example of this embodiment, the joining portion 52 is dented in the third direction DR3 or the fourth direction DR4 intersecting with the first direction DR1. For example, the joining portion 52 is dented in the direction towards a detection window 120 from the light blocking portion 20. Specifically, the joining portion 52 is dented in a direction towards the center of the detection window 120 from the outer circumference of the detection window 120. For example, in the sectional view of Fig. 5, the joining portion 52 is dented into the sectional shape of a concave shape. For example, in Fig. 5, the joining portion 52 includes a first joining surface and a second joining surface along the directions (DR3 and DR4) intersecting with the first direction DR1, and a third joining surface intersecting with (orthogonal to) the first joining surface and the second joining surface. In the sectional view of Fig. 5, the sectional shape of the first joining surface, the second joining surface, and the third joining surface of the joining portion 52 is a concave shape.

In addition, in a case of the second configuration example of Fig. 5, in the plan view of Fig. 2(B), the joining portion 52 is dented in the direction towards the light blocking portion 20 from the detection window 120 of the light transmitting portion 30. Specifically, in the detection window 120 having a circular shape, the joining portion 52 is dented in a direction towards the center from the outer circumference. Furthermore, it is not necessary that the joining portion 52 is dented in the entire position of the outer circumference of the detection window 120, and a region in which the joining portion 52 is not dented may be included in the outer circumference.

Further, specifically, in the configuration example of Fig. 4, the light transmitting portion 30 includes a convex portion 32 protruding in the direction towards the light blocking portion 20 from the detection window 120 of the light transmitting portion 30. That is, in the sectional view of Fig. 4, the light transmitting portion 30 includes the convex portion 32 of which the sectional shape is a convex shape. Then, the light blocking portion 20 includes a concave portion which is engaged with the convex portion 32 of the light transmitting portion 30. That is, in the sectional view of Fig. 4, the light blocking portion 20 includes the concave portion of which the sectional shape is a concave shape, and the concave portion is engaged with the convex portion 32 of the light transmitting portion 30.

On the other hand, in the second configuration example of Fig. 5, the light blocking portion 20 includes a convex portion 22 protruding in a direction towards the detection window 120 of the light transmitting portion 30 from the light blocking portion 20. That is, in the sectional view of Fig. 5, the light blocking portion 20 includes the convex portion 22 of which the sectional shape is a convex shape. Then, the light transmitting portion 30 includes a concave portion which is engaged with the convex portion 22 of the light blocking portion 20. That is, in the sectional view of Fig. 5, the light transmitting portion 30 includes the concave portion of which the sectional shape is a concave shape, and the concave portion is engaged with the convex portion 22 of the light blocking portion 20.

In addition, in this embodiment, the light transmitting portion 30 and the light blocking portion 20 are integrally formed. Specifically, the light transmitting portion 30 and the light blocking portion 20 are integrally formed by two-color molding or insert molding.

Here, the two-color molding and the insert molding are common in that the light transmitting portion 30 and the light blocking portion 20 are integrally formed by combining different materials. A difference is that in the two-color molding, a portion which becomes a primary side is molded, and then, a portion which becomes a secondary side is molded in the same mold by being integrated with the primary side, whereas in the insert molding, a portion which becomes a primary side is molded, and then, is taken out from a mold, and the taken out component is set in a mold on a secondary side, and is molded by being integrated with a portion which becomes a secondary side. Any molding method may be used as a method of integrally forming the light transmitting portion 30 and the light blocking portion 20, and in a case where mass production is assumed, the two-color molding is advantageous from the viewpoint that a primary side component is not required to be taken out from the mold. Furthermore, in this embodiment, the light transmitting portion 30 and the light blocking portion 20 are integrally formed, but it is not necessary that the two members are integrated with each other at a time point of forming each of the portions.

In addition, in this embodiment, it is desirable that the light transmitting portion 30 is formed of a material including a first resin material, and the light blocking portion 20 is also formed of the material including the first resin material. That is, the light transmitting portion 30 and the light blocking portion 20 are formed of the same material including the first resin material. More preferably, it is desirable that the light blocking portion 20 is formed of a material in which glass is contained in the first resin material (in a narrow sense, glass fiber) (a glass-containing resin material). Here, for example, polycarbonate, an ABS resin, an acrylic resin, or the like is able to be assumed as the first resin material. That is, the light transmitting portion 30 is able to be formed of polycarbonate, an ABS resin, or an acrylic resin, and the light blocking portion 20 is able to be formed of polycarbonate in which glass is contained, an ABS resin in which glass is contained, or an acrylic resin in which glass is contained.

That is, in this embodiment, the light blocking portion 20 is able to be formed of fiber reinforced plastics (FRP), and among them, it is particularly desirable that the light blocking portion 20 is formed of glass fiber reinforced plastics (GFRP) using glass fiber as fiber used in reinforcement. In GFRP, a thermoplastic resin may be used as a resin used along with the glass fiber, and in this embodiment, polycarbonate or an ABS resin is able to be used as the thermoplastic resin. In addition, a thermoplastic acrylic resin and a thermosetting acrylic resin are known as the acrylic resin, and in this embodiment, either the thermoplastic acrylic resin or the thermosetting acrylic resin is able to be used. GFRP is inexpensive in FRP, and is general, and thus, the light blocking portion 20 of this embodiment is able to be easily realized by adopting GFRP. Furthermore, various resin materials such as a polyester resin, a vinyl ester resin, an epoxy resin, and a phenolic resin are able to be used as a resin material in GFRP, and such resin materials are able to be widely used in the light blocking portion 20 according to this embodiment.

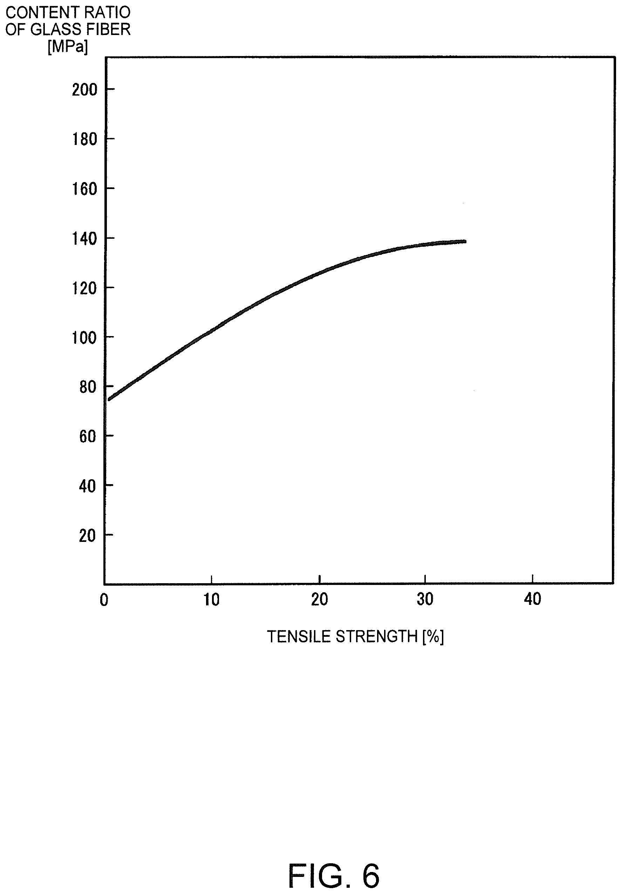

In Fig. 6, a graph illustrating a relationship between a content ratio of the glass fiber and a tensile strength of GFRP is illustrated. Fig. 6 is an example of a case where, in particular, polycarbonate is used as the resin material in GFRP. As it is obvious from Fig. 6, it is possible to increase a strength by containing glass, and as described above, a waterproof effect obtained by suppressing a modification or an effect of suppressing an impact on the inner component is able to be expected.

As described above, in this embodiment, it is desirable that the light transmitting portion 30 is formed of the resin material such as polycarbonate, and the light blocking portion 20 is also formed of the same resin material such as polycarbonate. More preferably, it is desirable that the light transmitting portion 30 is formed of the resin material such as polycarbonate, and the light blocking portion 20 is formed of a glass-containing resin material such as polycarbonate in which glass is contained. Then, by containing a colored (black or the like) dye in the light blocking portion 20, and thus, it is possible to block light. Furthermore, various modifications are able to be performed in which the light transmitting portion 30 is formed of an acrylic resin, the light blocking portion 20 is formed of an ABS resin, and the like. That is, the light transmitting portion 30 and the light blocking portion 20 may be formed of different resin materials.

Then, in the wrist-mountable biological information detecting device which is mounted on the arm, performs optical signaling with respect to a blood flow on the skin surface, and acquires biological information, the light transmitting portion 30 (a transparent member) transmitting light is necessary in order to accept an optical signal. Then, in a case where the shape of the light transmitting portion 30 is required to be complicated according to a restriction in the shape of the convex portion 40 of the light transmitting portion 30, the shape or the mounted state of a built-in component, or the like or a case where the biological information detecting device is required to be realized at a low cost, it is desirable that the light transmitting portion 30 is formed by using a transparent resin such as polycarbonate or an acrylic resin.

Then, in the biological information detecting device, it is necessary that waterproof performance is ensured such that sweat, water, or the like does not enter the device inner portion at the time of carrying the biological information detecting device. In order to ensure such waterproof performance, it is important to fix (join and immobilize) the light transmitting portion 30 and the light blocking portion 20 configuring the rear cover portion 304 by any method. For example, in a case where the light transmitting portion 30 is formed of a resin material, in general, a fixing method such as welding or bonding is used as a fixing method (a joining method, and an immobilizing method) of the light transmitting portion 30 and the light blocking portion 20.

However, in the fixing method using the welding, peeling of the light transmitting portion 30 and the light blocking portion 20 due to an impact or the like at the time of carrying or dropping the biological information detecting device, peeling due to a modification or the like of the rear cover portion 304 caused by water in a pressure test or the like may occur. In the fixing method using the bonding, the same problem may occur, and thus, it is difficult to ensure fixing quality in which the light transmitting portion 30 and the light blocking portion 20 are immobilized for a long period of time.

From this viewpoint, in this embodiment, in the wrist-mountable biological information detecting device performing optical biological sensing, a method is adopted in which the light transmitting portion 30 disposed on the test body side (the arm side) is formed of a resin material, and is fixed (joined) to the light blocking portion 20 by the two-color molding or the insert molding. That is, in this embodiment, the light transmitting portion 30 which is a transparent member and the light blocking portion 20 which is a colored member are integrally formed by the two-color molding, the insert molding, or the like, and thus, the light transmitting portion 30 and the light blocking portion 20 are fixed to each other. In a case where the light transmitting portion 30 and the light blocking portion 20, for example, are formed of the same material including a resin material (polycarbonate or the like), the light transmitting portion 30 and the light blocking portion 20 are formed of materials of which the melting points are identical to each other or close to each other. Accordingly, at the time of molding a secondary resin (for example, the light blocking portion 20) in the two-color molding or the insert molding, a boundary surface of a primary resin (for example, the light transmitting portion 30) is dissolved by heat, and thus, the light transmitting portion 30 and the light blocking portion 20 are reliably fixed to each other. That is, even though a bonding agent or the like is not used, the light transmitting portion 30 and the light blocking portion 20 are reliably fixed to each other, and thus, waterproof performance is able to be improved.

For example, in the related art of PTL 1 described above, a structure is adopted in which the sensor unit of the biological information is retained by the retaining unit formed of synthetic rubber or the like, and the device main body, the pulse wave sensor unit, and the retaining unit have waterproof properties, and thus, waterproof performance of the biological information detecting device is realized. However, in the related art, the method is adopted in which the sensor unit is retained by the retaining unit formed of synthetic rubber or the like, and thus, the structure of the device becomes complicated, the number of components increases, a cost increase in the device is caused, or thinning of the device is disturbed.

From this viewpoint, according to this embodiment, the rear cover portion 304 of the case portion 300 is able to be formed by a simple structure including a small number of components in which the light transmitting portion 30 and the light blocking portion 20 are integrally formed and fixed to each other. Accordingly, it is possible to realize reliable waterproof performance of the biological information detecting device at a low cost and to easily realize thinning and downsizing of the device.

In addition, in this embodiment, as illustrated in Fig. 4 and Fig. 5, a structure is adopted in which the joining portion 52 between the light transmitting portion 30 and the light blocking portion 20 protrudes or is dented along the directions (DR3 and DR4) intersecting with the first direction DR1 towards the biological information detecting device from the test body. By adopting such a structure, it is possible to ensure a wide area as a welding portion of the joining portion 52 between the light transmitting portion 30 and the light blocking portion 20, to improve fixing performance between the light transmitting portion 30 and the light blocking portion 20, and to further improve waterproof performance. In addition, as described above, the joining portion 52 protrudes or is dented along the direction intersecting with the first direction DR1, and thus, the structure of the joining portion 52 becomes a structure in which concavities and convexities are combined. For example, in Fig. 4, a structure is obtained in which the convex portion 32 of the light transmitting portion 30 and the concave portion of the light blocking portion 20 are combined, and in Fig. 5, a structure is obtained in which the convex portion 22 of the light blocking portion 20 and the concave portion of the light transmitting portion 30 are combined. Accordingly, even in a case where an external stress acts, or the device is modified due to the external stress, the light transmitting portion 30 is not easily peeled off from the light blocking portion 20 by the structure in which the concavities and convexities are combined, and thus, a structure is able to be easily realized in which the light transmitting portion 30 is not physically detached from the light blocking portion 20.

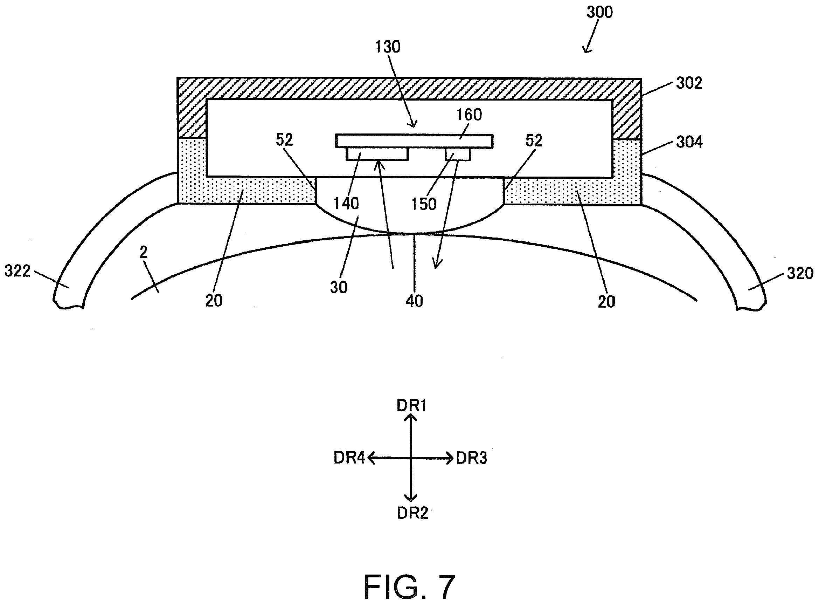

For example, in Fig. 7, a configuration example of a biological information detecting device of a comparative example of this embodiment is illustrated. In the comparative example of Fig. 7, the joining portion 52 between the light transmitting portion 30 and the light blocking portion 20 does not protrude or is not dented in the direction intersecting with the first direction DR1. The joining portion 52 does not have a structure of concavities and convexities, and the joining portion 52 includes only a joining surface along the first direction DR1, and does not include a joining surface along the direction intersecting with the first direction DR1. Accordingly, for example, in a case where an external force acts along the first direction DR1, fixing performance becomes weak on the joining surface along the first direction DR1, or the light transmitting portion 30 is peeled off from the light blocking portion 20, and thus, may be detached therefrom. In particular, as described below, an external force caused by suppressing the pressing force acts in the vicinity of the joining portion 52, and the light transmitting portion 30 is easily detached from the light blocking portion 20 due to the external force. In addition, in the comparative example of Fig. 7, the joining portion 52 only includes the joining surface along the first direction DR1, and thus, sweat or moisture easily enters through the joining surface, and waterproof performance may decrease.

From this viewpoint, in the biological information detecting device of this embodiment of Fig. 4 and Fig. 5, the structure of the joining portion 52 is a structure in which the joining portion 52 protrudes or is dented in the direction intersecting with the first direction DR1, and the joining portion 52 includes the first joining surface, the second joining surface, and the like along the directions (DR3 and DR4) intersecting with the first direction DR1. Accordingly, for example, even in a case where an external force along the first direction DR1 acts, for example, it is possible to effectively prevent the light transmitting portion 30 from being detached from the light blocking portion 20 by including the first joining surface and the second joining surface described above in the joining portion 52. In addition, the joining portion 52 has a structure in which the joining portion 52 includes the first joining surface and the second joining surface, and the third joining surface intersecting with the first joining surface and the second joining surface, and thus, for example, it is possible to ensure a wide area as the welding portion of the joining portion 52 in the two-color molding or the insert molding. Accordingly, it is possible to improve fixing performance between the light transmitting portion 30 and the light blocking portion 20 or waterproof performance of the joining portion 52, and for example, it is possible to maintain the fixing performance or the waterproof performance for a long period of time.

In addition, in a case where the case portion 300 has a structure which is easily modified, a path through which a liquid or moisture vapor flows is generated due to the modification, or a pressure from the outside is imparted to the inner component. Here, a gap between the light transmitting portion 30 and the light blocking portion 20 or the like is considered as an inflow path. For example, even in a case where the light transmitting portion 30 and the light blocking portion 20 are formed of a common resin base by the two-color molding or the insert molding, only a small amount of the surface is dissolved and welded, and thus, for example, in the structure of the comparative example of Fig. 7, there is a possibility in which a liquid or moisture vapor enters a small gap between the light transmitting portion 30 and the light blocking portion 20. In addition, a pressure from the outside is imparted to the inner component due to the modification described above, and thus, the inner component may be broken or the like.

From this viewpoint, in this embodiment, the joining portion 52 between the light transmitting portion 30 and the light blocking portion 20 has a structure in which the concavities and convexities are combined, and thus, a modification due to a shift or the like in the joining portion 52 rarely occurs. Accordingly, it is also possible to effectively suppress deterioration in waterproof performance, a breakage in the inner component, or the like due to the modification. In addition, in a case where the joining portion 52 between the light transmitting portion 30 and the light blocking portion 20 has a structure in which concavities and convexities are combined, for example, thinning and downsizing of the device are also easily realized, compared to the method of PTL 1 described above in which the sensor unit is retained by the retaining unit formed of synthetic rubber.

In particular, in this embodiment, the light blocking portion 20 is formed of a glass-containing resin material, and thus, the light blocking portion 20 becomes a member which is rarely modified. For this reason, it is not necessary to increase the thickness of the light blocking portion 20, and it is possible to realize thinning and weight saving of the biological information detecting device while increasing waterproof performance and strength. Furthermore, the light transmitting portion 30 may be also formed of the glass-containing resin material.

Further, by thinning the biological information detecting device, an effect is also able to be expected in which detection accuracy or the like of the biological information increases (a decrease in accuracy is suppressed). This is because in a case where the biological information detecting device is thick, the biological information detecting device is in contact with a sleeve during a period of wearing long-sleeved clothes, and thus, the device itself swings according to the movement of the sleeve. It is preferable that a biological sensor such as a pulse wave sensor is used by adhering to the skin, but in a case where the device swings, floating occurs, and thus, measurement accuracy may decrease. From this viewpoint, in a case where the biological information detecting device is thinned, it is possible to suppress the floating of the device which occurs by being in contact with the sleeve or the like, and to increase detection accuracy or the like.

### 3. Specific Structure Example

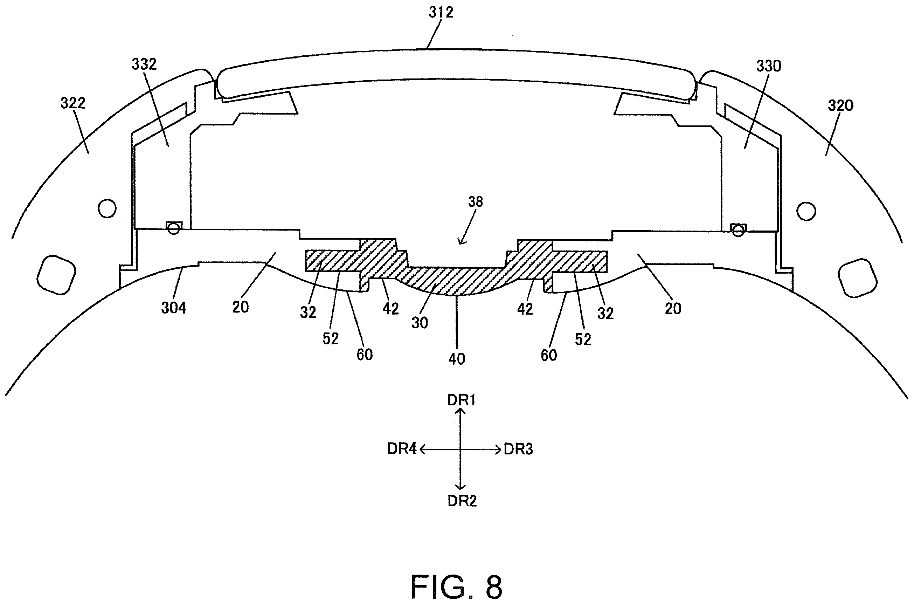

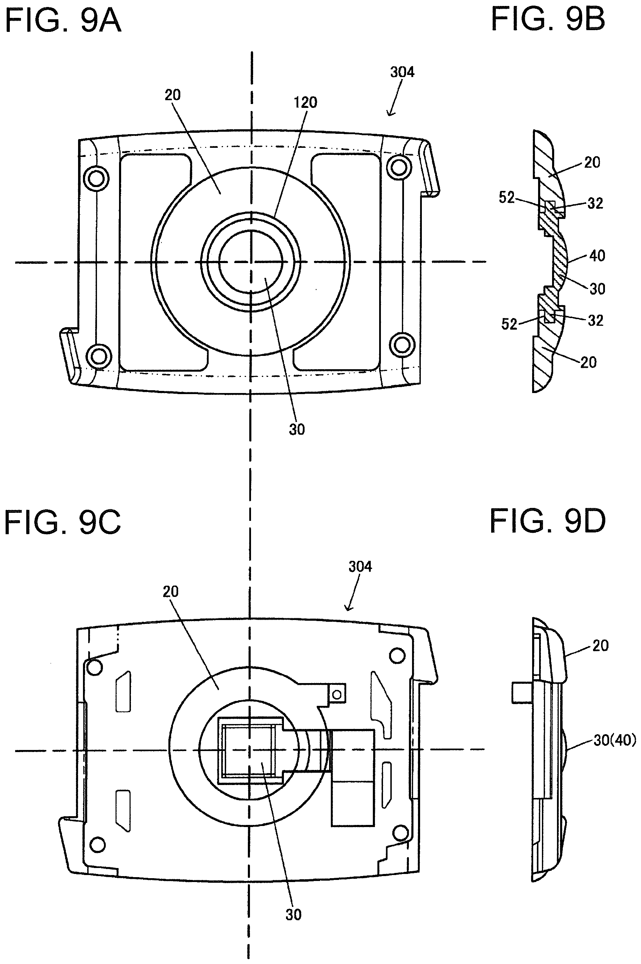

Fig. 8 is a sectional view illustrating a specific structure example of the biological information detecting device, and Fig. 9(A) to Fig. 9(D) are plan views and a side view illustrating a specific structure example of the rear cover portion 304. Fig. 9 is a sectional view in which a structure in the vicinity of the sensor unit 130 of Fig. 8 is enlarged.

As illustrated in Fig. 8, in this embodiment, the joining portion 52 between the light transmitting portion 30 and the light blocking portion 20 has a structure in which the joining portion 52 protrudes along the direction intersecting with the first direction DR1 (or a structure in which the joining portion 52 is dented). That is, the rear cover portion 304 having a structure illustrated in Fig. 9(A) to Fig. 9(D) is formed by the light transmitting portion 30 and the light blocking portion 20 which are joined to each other by the joining portion 52 having such a structure.

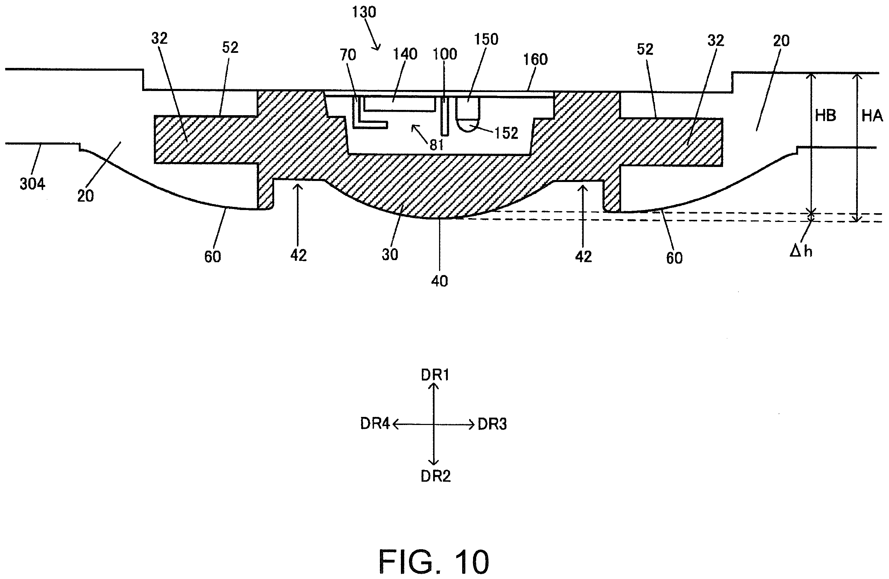

In addition, as illustrated in Fig. 8, a convex portion 40 is formed on the surface of the light transmitting portion 30 on the test body side, and a concave portion 38 is formed on the surface of the light transmitting portion 30 on a side opposite to the test body. As illustrated in Fig. 10, the sensor unit 130 is attached to the position of the concave portion 38. That is, the light emitting unit 150, the light receiving unit 140, and the light blocking member 70 are mounted on the substrate 160, and the substrate 160 is attached to the position of the concave portion 38 of the light transmitting portion 30. Furthermore, hereinafter, a case where the number of light emitting units 150 and the number of light receiving units 140 are respectively one will be described, but this embodiment is not limited thereto. For example, a plurality of light receiving units (for example, a first light receiving unit and a second light receiving unit) may be disposed corresponding to one light emitting unit. Alternatively, a plurality of light emitting units may be disposed.

A dome type lens 152 (in a broad sense, a condensing lens) disposed in the light emitting unit 150 is a lens for collecting light from an LED chip (in a broad sense, a light emitting element chip) which is resin-sealed (sealed with light transmitting resin) in the light emitting unit 150. That is, in the chip package type (surface mounting type) light emitting unit 150, the LED chip is disposed under the dome type lens 152, and the light from the LED chip is collected by the dome type lens 152 and is emitted to the test body. Accordingly, it is possible to increase an emission efficiency of the light with respect to the test body, and to increase detection sensitivity of the sensor unit 130.

The light blocking member 70 is a member for blocking light. For example, in Fig. 10, the light blocking member 70 blocks light with respect to the light receiving unit 140. That is, the light blocking member 70 is not disposed on the light emitting unit 150 side, but is disposed on the light receiving unit 140 side. For example, the light blocking member 70 is disposed to surround (to cover) the light receiving unit 140, and blocks light incident on the light receiving unit 140, but does not block light with respect to the light emitting unit 150. Here, a modification is also able to be performed in which the light blocking member 70 is disposed on the light emitting unit 150 side.

It is desirable that at least an inner side surface of the light blocking member 70 is subjected to antireflection processing. For example, the color of the surface of the light blocking member 70 (the inner side surface or the like) is set to a predetermined color such as black, and thus, prevents scattered reflection of light. Alternatively, the surface of the light blocking member 70 may have a moth-eye structure. For example, an antireflection structure is obtained by forming a concave and convex structure having a period of dozens to hundreds nm on the surface. According to such antireflection processing, for example, it is possible to effectively prevent reflected light on the surface of the light blocking member 70 from becoming stray light, and thus, from becoming a noise component of a detection signal.

The light blocking member 70, for example, is formed by performing sheet-metal processing with respect to metal (for example, an alloy of tin and copper). For example, one metal plate is subjected to sheet-metal processing, and thus, the light blocking member 70 is formed. Then, the light blocking member 70 includes the light blocking wall 100 which is disposed between the light emitting unit 150 and the light receiving unit 140. The light blocking wall 100 blocks light (direct light or the like) from the light emitting unit 150 from being incident on the light receiving unit 140. Among a plurality of metal surfaces configuring the light blocking member 70, the light blocking wall 100 is realized by a first metal surface disposed between the light emitting unit 150 and the light receiving unit 140. The light blocking member 70 includes a second metal surface and a third metal surface which intersect with (are orthogonal to) the first metal surface, and become metal surfaces of side surfaces of the light blocking member 70. In addition, the light blocking member 70 includes a fourth metal surface which intersects with (is orthogonal to) the first metal surface, the second metal surface, and the third metal surface, and becomes a metal surface of a top surface of the light blocking member 70. In addition, the light blocking member 70 includes a fifth metal surface which intersects with (is orthogonal to) the second metal surface, the third metal surface, and the fourth metal surface, and becomes a metal surface of a rear surface of the light blocking member 70.

For example, an optical efficiency and optical performance of the sensor unit 130 of the biological information detecting device are improved as a distance between the light emitting unit 150 and the light receiving unit 140 becomes shorter. For example, the optical efficiency and the optical performance decrease in inverse proportion to the square of the distance. Accordingly, it is desirable that the distance between the light emitting unit 150 and the light receiving unit 140 becomes shortest.

On the other hand, in a case where the distance between the light emitting unit 150 and the light receiving unit 140 is excessively short, direct light from the light emitting unit 150 is incident on the light receiving unit 140, and thus, a DC component increases or the like, and performance decreases. For this reason, in this embodiment, the light blocking wall 100 is disposed between the light emitting unit 150 and the light receiving unit 140.

In this case, a method of forming the light blocking member 70 by injection molding is considered. A method using the injection molding is an advantageous method from the viewpoint of mass productivity of the device or the like.

However, in a case where the light blocking member 70 is formed by the injection molding, a wall thickness of the light blocking wall 100 becomes thick. That is, in a case where the wall thickness of the light blocking wall 100 is designed to be thin, the portion of the light blocking wall 100 is not sufficiently filled with a resin at the time of performing the injection mold, and thus, the light blocking wall 100 having sufficient strength is not able to be realized. For this reason, in the method using the injection molding, the thickness of the light blocking wall 100, for example, becomes greater than or equal to 0.4 mm.

Then, in a case where the light blocking wall 100 becomes thick as described above, the distance between the light emitting unit 150 and the light receiving unit 140 also becomes long. Accordingly, for example, a light path length through the test body between the light emitting unit 150 and the light receiving unit 140 also becomes long, and thus, the optical efficiency and the optical performance of the sensor unit 130 decrease.

Therefore, in Fig. 10, the light blocking member 70 is formed by performing sheet-metal processing with respect to metal. For example, one metal plate is bent by performing the sheet-metal processing, and thus, the light blocking member 70 is formed. Then, a light blocking surface facing the light emitting unit 150 is the light blocking wall 100 blocking the direct light from the light emitting unit 150 from being incident on the light receiving unit 140.

Thus, in a case where the light blocking wall 100 is realized by the sheet-metal processing, it is possible to thin the light blocking wall, compared to the method using the injection molding. For example, in a case where the sheet-metal processing is used, the light blocking member 70 having sufficient strength is able to be realized even in a case where the thickness of the metal surface, for example, is approximately 0.1 mm. For this reason, the thickness of the light blocking wall 100, for example, is also able to be approximately 0.1 mm. Accordingly, it is possible to sufficiently thin the light blocking wall 100, and according to this, it is also possible to shorten the distance between the light emitting unit 150 and the light receiving unit 140, compared to the method using the injection molding in which the thickness of the light blocking wall, for example, becomes greater than or equal to 0.4 mm. Accordingly, it is also possible to shorten the light path length of light through the test body from the light emitting unit 150 to the light receiving unit 140 while preventing the direct light from the light emitting unit 150 from being incident on the light receiving unit 140 by the light blocking wall 100, and thus, it is possible to improve detection performance of the sensor unit 130, or the like.

In addition, an opening portion 81 is formed in the metal surface of the top surface of the light blocking member 70 (the fourth metal surface). That is, the opening portion 81 is formed in the upper portion of the light receiving unit 140. The opening portion 81 functions as a diaphragm unit which diaphragms light from the object (reflected light or the like) in the light path between the test body (the object) and the light receiving unit 140.

For example, in the biological information detecting device of this embodiment, the surface of the light transmitting portion 30 in contact with the skin which is the test body becomes a contact surface having a limited area. Then, for example, a relatively soft object such as a skin is brought into contact with the contact surface having a limited area of the light transmitting portion 30 formed of a hard material such as a resin or glass. Then, a region which is not in contact with the skin or a region having a weak contact pressure is formed in the vicinity of a peripheral edge portion (an outer circumference portion) of the light transmitting portion 30 from the viewpoint of elastic dynamics. In addition, even when a case where an external force is imparted to the biological information detecting device, and thus, a moment is generated in the device, and the like, the region in the vicinity of the peripheral edge portion of the contact surface most easily floats. Light in such a region easily optically strengthens or weakens according to a change in a dynamic contact state. Then, in a case where such light is incident on the light receiving unit 140 or the like, the light becomes a noise irrelevant to a pulse component.

In addition, even in a static contact state, signal integrity is able to decrease. In a case where the surface of the light transmitting portion 30 is not accurately in contact with the skin, external light which is not emitted from the light emitting unit 150 is incident on the light receiving unit 140 or the like. On the other hand, in a case where an excessive contact pressure is imparted, a subcutaneous blood vessel is crushed, and thus, it is difficult for a beat component to be input into light passing through the region.

Signal integrity of a pulse wave detection signal decreases as such a noise is considerably superimposed on the light, and in various biological measurements on the pulse monitor side or the like, reliability of measurement data decreases.

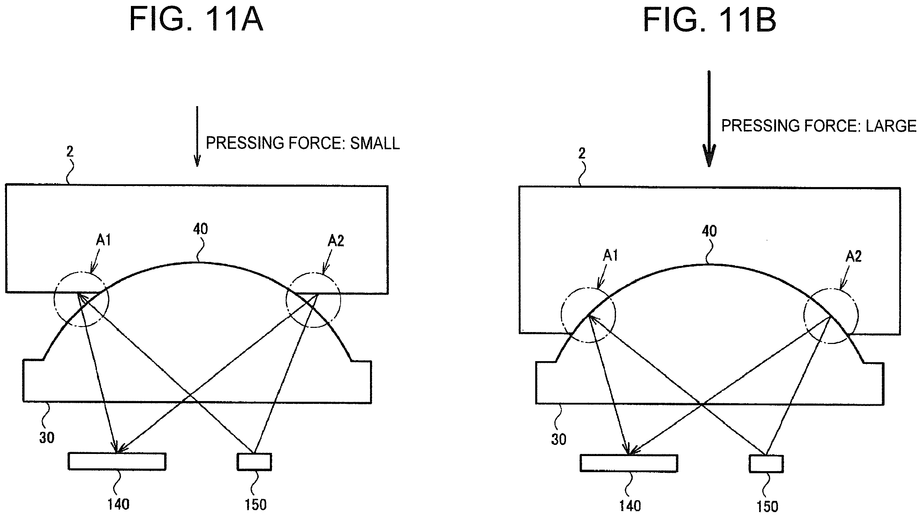

For example, Fig. 11(A) illustrates a case where a pressing force of the convex portion 40 of the light transmitting portion 30 which is imparted to a skin 2 which is the test body is small, and Fig. 1(B) illustrates a case where the pressing force is large. Focusing on locations denoted by A1 and A2 of Fig. 11(A) and Fig. 11(B), a contact state between the skin 2 and the convex portion 40 is changed according to a change in the pressing force. For example, in Fig. 11 (A), in the locations of A1 and A2, the skin 2 and the convex portion 40 are in a non-contact state or a weak-contact state, and in Fig. 11(B), the skin 2 and the convex portion 40 are in a contact state. Accordingly, intensity of light which is emitted from the light emitting unit 150 and returns to the light receiving unit 140, or the like is changed in Fig. 11 (A) and Fig. 11(B), and thus, reliability of measurement data decreases. That is, in the locations denoted by A1 and A2 of Fig. 11(A) and Fig. 11(B), the pressing force on the contact surface is rapidly changed according to a minor change in a load, and thus, the reliability of the measurement data considerably decreases.

For example, in Fig. 11(A) and Fig. 11(B), the contact surface of the light transmitting portion 30 in contact with the skin of a human body is configured to have a convex shape (a convex portion) of a curved surface shape. Thus, a degree of adhesion of the light transmitting portion 30 with respect to the skin surface is improved, and thus, it is possible to prevent noise light such as reflected light from the skin surface or disturbance light from entering the light transmitting portion 30.

However, in the peripheral edge portion having a convex shape (the outer circumference portion), a contact pressure with respect to the skin relatively decreases compared to the center portion. In this case, in a case where a contact pressure in the center portion is optimized, a contact pressure in the peripheral edge portion is less than an optimal range. On the other hand, in a case where the contact pressure in the peripheral edge portion is optimized, the contact pressure in the center portion is greater than the optimal range.

In a case where the contact pressure is less than the optimal range, the pulse wave sensor is in contact with the skin or is separated from the skin according to the swing of the device, even though the pulse wave sensor is in contact with the skin, the vein is not suppressed by the pulse wave sensor, and the like, and thus, a body motion noise is superimposed on the pulse wave detection signal. In a case where the noise component is reduced, it is possible to obtain a pulse wave detection signal having a higher M/N ratio (S/N ratio). Here, M indicates a signal level of the pulse wave detection signal, and N indicates a noise level.

In order to solve such a problem, in Fig. 10, the opening portion 81 is disposed in the top surface of the light blocking member 70. That is, the opening portion 81 which functions as the diaphragm unit is disposed such that the light (stray light) in the locations denoted by A1 and A2 of Fig. 11 (A) and Fig. 11(B) is not detected, and diaphragms the light. For example, light passing through the center portion (for example, the apex of the convex portion) of the light transmitting region of the light transmitting portion 30 in which a pressing force is optimized is maximally transmitted without being blocked, whereas light passing through the vicinity of the peripheral edge portion of the light transmitting region (for example, the convex portion) of the light transmitting portion 30 is blocked. Thus, as illustrated in Fig. 11(A) and Fig. 11(B), even in a case where the contact state is changed in the locations denoted by A1 and A2, the state of the light in the locations denoted by A1 and A2 is not affected by a light receiving result. Accordingly, it is possible to improve reliability of measurement data, or the like.

In addition, in Fig. 10, the light transmitting portion 30 includes a first convex portion 40 protruding in the direction (the second direction DR2) opposite to the first direction DR1. In addition, a second convex portion 60 is disposed to surround the light transmitting portion 30 (for example, the first convex portion 40 and the detection window 120). Herein, the first convex portion 40 will be suitably referred to as the convex portion 40, and the second convex portion 60 will be suitably referred to as a pressing force suppressing unit 60. Specifically, in Fig. 10, the pressing force suppressing unit 60 (in other words, a second convex portion, and the same hereinafter) suppressing the pressing force imparted to the test body (the skin of the wrist) by the convex portion 40 (the first convex portion) of the light transmitting portion 30 is disposed in the light blocking portion 20. In Fig. 10, the pressing force suppressing unit 60 (the second convex portion) is disposed to surround the convex portion 40 (the first convex portion) of the light transmitting portion 30. Specifically, the surface of the light blocking portion 20 (the cover member) functions as the pressing force suppressing unit 60. That is, the pressing force suppressing unit 60 is formed by molding the surface of the light blocking portion 20 around the convex portion 40 of the light transmitting portion 30 (the surface around the detection window) into the shape of a bank.

Then, in a case where the height of the convex portion 40 (the first convex portion) in the second direction DR2 towards the test body from the biological information detecting device is set to HA, the height of the pressing force suppressing unit 60 (the second convex portion) is set to HB, and a value obtained by subtracting the height HB from the height HA (a difference between the height HA and the height HB) is set to Δh, a relationship of Δh = HA - HB > 0 is established. For example, the convex portion 40 protrudes from the pressing force suppressing surface of the pressing force suppressing unit 60 to the test body side such that Δh > 0 is satisfied.

Thus, by disposing the convex portion 40 in which Δh > 0 is satisfied, for example, it is possible to impart an initial pressing force for exceeding a vein vanishing point to the test body. In addition, by disposing the pressing force suppressing unit 60 for suppressing the pressing force imparted to the test body by the convex portion 40, it is possible to suppress a variation in the pressing force to be minimized in a use range where the biological information is measured by the biological information detecting device, and to reduce the noise component or the like. Here, the vein vanishing point indicates a point where a signal which is generated from the vein and is superimposed on the pulse wave signal vanishes, or decreases to the extent of not affecting pulse wave measurement when the convex portion 40 is brought into contact with the test body, and the pressing force gradually strengthens.

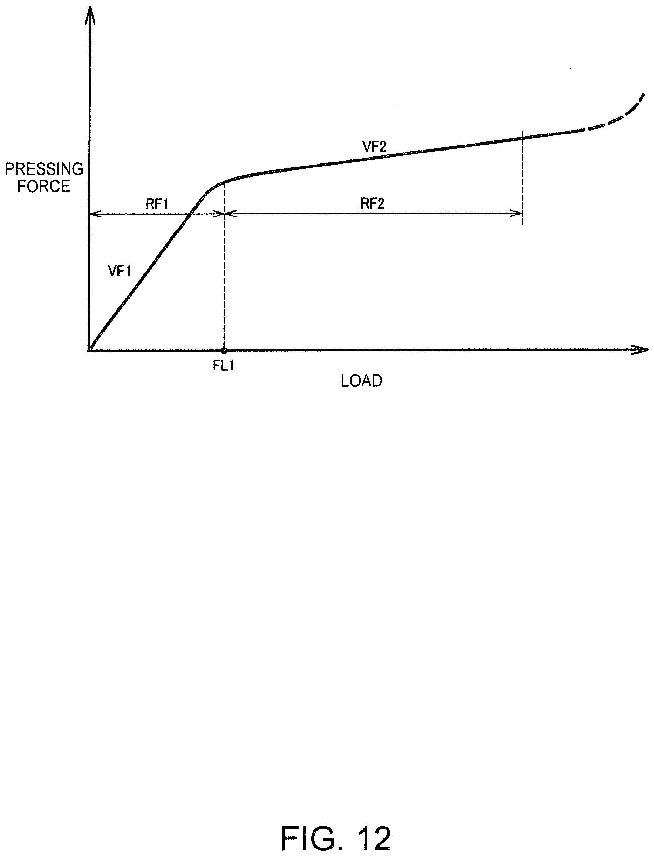

Specifically, as illustrated in Fig. 12, the pressing force suppressing unit 60 (the second convex portion) suppresses the pressing force imparted to the test body by the convex portion 40 (the first convex portion) such that a pressing force change amount VF2 in a second load range RF2 where the load of the load mechanism is greater than FL1 becomes smaller than a pressing force change amount VF1 in a first load range RF1 where the load of the load mechanism (the band portion or the like) of the biological information detecting device becomes 0 to FL1. That is, the pressing force change amount VF1 increases in the first load range RF1 which is an initial pressing force range, whereas the pressing force change amount VF2 decreases in the second load range RF2 which is a use range of the biological information detecting device.

That is, in the first load range RF1, the pressing force change amount VF1 increases, and an inclination of change properties of the pressing force with respect to the load increases. Such a pressing force having a large inclination of the change properties is realized by Δh corresponding to a projecting amount of the convex portion 40. That is, by disposing the convex portion 40 in which Δh > 0 is satisfied, even in a case where the load of the load mechanism is small, it is possible to impart a sufficient initial pressing force necessary for exceeding the vein vanishing point to the test body.

On the other hand, in the second load range RF2, the pressing force change amount VF2 decreases, and the inclination of change properties of the pressing force with respect to the load decreases. Such a pressing force having a small inclination of the change properties is realized by suppressing the pressing force by the pressing force suppressing unit 60. That is, the pressing force imparted to the test body by the convex portion 40 is suppressed by the pressing force suppressing unit 60, and thus, in the use range of the biological information detecting device, it is possible to suppress a variation in the pressing force to be minimized even in a case where the load varies, and the like. Accordingly, the noise component is reduced, and the like.

Then, as illustrated in Fig. 10, in this embodiment, the joining portion 52 protrudes along the directions (DR3 and DR4) intersecting with (orthogonal to) the first direction DR1 on the first direction DR1 side of the pressing force suppressing unit 60 (the second convex portion). Alternatively, as illustrated in the example of Fig. 5, the joining portion 52 may be dented along the directions (DR3 and DR4) intersecting with (orthogonal to) the first direction DR1 on the first direction DR1 side of the pressing force suppressing unit 60 (the second convex portion).

That is, as described above, the light blocking portion 20 is provided with the pressing force suppressing unit 60 suppressing the pressing force imparted to the test body (the skin of the wrist) by the convex portion 40 of the light transmitting portion 30. Accordingly, when the convex portion 40 imparts the pressing force to the test body, an external force for suppressing the pressing force is imparted to the region of the pressing force suppressing unit 60 of the light blocking portion 20. Specifically, the external force along the first direction DR1 which is a direction towards the biological information detecting device from the test body is imparted to the pressing force suppressing unit 60 as the external force caused by suppressing the pressing force.

In this case, in a case where the joining portion 52, for example, has a structure as illustrated in the comparative example of Fig. 7, the light transmitting portion 30 is peeled off from the light blocking portion 20 by the external force caused by suppressing the pressing force. That is, in a case where the external force along the first direction DR1 caused by suppressing the pressing force is imparted to the joining portion 52, fixing performance on the joining surface along the first direction DR1 weakens, and thus, the light transmitting portion 30 is detached from the light blocking portion 20. In addition, the fixing performance weakens, and thus, waterproof performance also decreases.

From this viewpoint, in this embodiment, as illustrated in Fig. 10, the joining portion 52 protrudes (or is dented) along the directions (DR3 and DR4) intersecting with the first direction DR1 on the first direction DR1 side of the pressing force suppressing unit 60. Accordingly, even in a case where the external force along the first direction DR1 is imparted as the external force caused by suppressing the pressing force in the pressing force suppressing unit 60, it is possible to prevent the light transmitting portion 30 from being peeled off from the light blocking portion 20. That is, in Fig. 10, the joining portion 52 has a concave and convex structure, and includes the first joining surface and the second joining surface along the direction intersecting with the first direction DR1. Accordingly, even in a case where the external force along the first direction is imparted in the vicinity of the pressing force suppressing unit 60, the joining portion 52 includes the first joining surface and the second joining surface along the direction intersecting with the first direction DR1 which is the direction of the external force, and thus, it is possible to effectively prevent the light transmitting portion 30 from being detached from the light blocking portion 20. Accordingly, the pressing force imparted to the test body by the convex portion 40 is suppressed by the pressing force suppressing unit 60, and thus, it is also possible to effectively prevent the light transmitting portion 30 from being peeled off from the light blocking portion 20 due to the external force caused by suppressing the pressing force while suppressing a variation in the pressing force to be minimized, reducing the noise component, and the like.

In addition, in Fig. 10, a groove portion 42 is disposed between the convex portion 40 (the first convex portion) of the light transmitting portion 30 and the pressing force suppressing unit 60 (the second convex portion). For example, the groove portion 42 is disposed around the convex portion 40 of the light transmitting portion 30. The height of a bottom surface of the groove portion 42 is lower than the height of the pressing force suppressing surface (the height in the highest end portion) of the pressing force suppressing unit 60, and the bottom surface of the groove portion 42 is a surface positioned lower than the pressing force suppressing surface of the pressing force suppressing unit 60 (on the sensor unit 130 side).

For example, in a case where a relatively soft object such as a skin is brought into contact with the contact surface of the light transmitting portion 30 formed of a hard material such as a resin or glass, a region which is not in contact with the skin or region having a weak contact pressure is formed in the vicinity of the peripheral edge portion (the outer circumference portion) of the light transmitting portion 30. Accordingly, for example, in a case where a flat portion is formed without disposing the groove portion 42 as illustrated in Fig. 10 around the convex portion 40, the flat portion is not in contact with the skin, the contact state becomes a weak-contact state, or the like, and thus, the contact state is dynamically changed. Then, light easily optically strengthens or weakens due to such a dynamic change of the contact state, and in a case where such light is incident on the light receiving unit 140, the light becomes a noise irrelevant to a pulse component.

From this viewpoint, by disposing the groove portion 42 as illustrated in Fig. 10, it is possible to effectively prevent a region in which the contact state is dynamically changed from being formed, and thus, signal integrity is improved, and the like. That is, the bottom surface of the groove portion 42 and the test body are in the non-contact state, and thus, the dynamic change of the contact state rarely occurs, and detection performance is able to be improved.

### 4. Suppression of Modification

Fig. 13 is a sectional view illustrating a modification example of the biological information detecting device of this embodiment. In the modification example, third convex portions 360, 361, 362, and 363 (back-up members) are disposed in the case portion 300. Specifically, the third convex portions 360, 361, 362, and 363 are disposed between the circuit case 352 supporting the circuit substrate 350 and the light transmitting portion 30. For example, the third convex portions 360, 361, 362, and 363 are disposed in the shape where the third convex portions 360, 361, 362, and 363 protrude from the lower surface of the circuit case 352 to the second direction DR2 side (the light transmitting portion 30 side). The third convex portions 360, 361, 362, and 363, for example, function as a member preventing at least one of the light transmitting portion 30 and the light blocking portion 20 from being modified in the first direction DR1 in the joining portion 52. Furthermore, herein, the third convex portions 360, 361, 362, and 363 will be suitably referred to as modification suppressing units 360, 361, 362, and 363. Here, the shape of the modification suppressing units 360, 361, 362, and 363 is not limited to a convex shape.

For example, in Fig. 13, the display panel 314, the circuit substrate 350, the circuit case 352, and the panel frame 354 are disposed in the case portion 300 as the inner component (the module).

The display panel 314 is a panel displaying various information items such as a pulse rate, calory consumption, a time, and the like, and for example, is realized by a liquid crystal display panel (LCD) or the like. The display panel 314 is disposed by being supported by the panel frame 354. The circuit substrate 350 is a substrate on which a device such as the processing unit 200, a storage unit 240, and a communication unit 250 of Fig. 15 and Fig. 16 described below is mounted. That is, the circuit substrate 350 is a main substrate (a rigid substrate) of the biological information detecting device. The circuit case 352 is a case member which is disposed to support the circuit substrate 350. For example, a battery or the like is able to be disposed in the circuit case 352.

In Fig. 13, the modification suppressing units 360, 361, 362, and 363 (third convex portion) are disposed between such a module formed of the display panel 314, the circuit substrate 350, the circuit case 352, and the panel frame 354 and the rear cover portion 304. Then, the external force along the first direction DR1 acts, and thus, the rear cover portion 304 (at least one of the light transmitting portion 30 and the light blocking portion 20) is modified on the first direction DR1 side. In this case, the modification suppressing units 360, 361, 362, and 363 press, for example, the rear cover portion 304 on the second direction DR2 side which is the direction opposite to the first direction DR1, and thus, support the rear cover portion 304, and suppress the modification of the rear cover portion 304.

For example, in a case where the biological information detecting device is used by being mounted on the wrist or the like of the user, the external force along the first direction DR1 acts in the convex portion 40 or the pressing force suppressing unit 60 of the light transmitting portion 30, and the rear cover portion 304 is modified due to the external force. That is, the light transmitting portion 30 or the light blocking portion 20 of the rear cover portion 304 is modified on the first direction DR1 side. Then, in a case where such a modification occurs over a long period of time, fixing performance of the joining portion 52 decreases, and the light transmitting portion 30 may be detached from the light blocking portion 20, and the like.

From this viewpoint, in Fig. 13, such a modification of the rear cover portion 304 (the light transmitting portion 30 and the light blocking portion 20) on the first direction DR1 side is suppressed by the modification suppressing units 360, 361, 362, and 363. Accordingly, it is possible to maintain the fixing performance of the joining portion 52 over a long period of time.

Furthermore, the modification suppressing units 360, 361, 362, and 363, for example, may be formed of an elastic material. For example, in a case where the rear cover portion 304 is modified on the first direction side DR1, displacement due to this modification is imparted to the display panel 314, and thus, display abnormality such as moire may occur in the display panel 314. From this viewpoint, in a case where the modification suppressing units 360, 361, 362, and 363 are formed of an elastic material or the like, the displacement due to such a modification is prevented from being imparted to the display panel 314, and thus, it is possible to prevent the display abnormality as described above from occurring.

In addition, in Fig. 13, the modification suppressing units 361 and 362 are disposed in the vicinity of the joining portion 52 between the light transmitting portion 30 and the light blocking portion 20. Thus, it is possible to more effectively prevent the fixing performance of the joining portion 52 between the light transmitting portion 30 and the light blocking portion 20 from decreasing due to the external force along the first direction DR1, and thus, the light transmitting portion 30 from being detached from the light blocking portion 20. In particular, in Fig. 13, in a case where the pressing force suppressing unit 60 is disposed around the convex portion 40 of the light transmitting portion 30, the modification suppressing units 361 and 362 are disposed in the vicinity of the position of the pressing force suppressing unit 60 on the first direction DR1 side. Accordingly, even in a case where external force caused by suppressing the pressing force by the pressing force suppressing unit 60 is imparted to the pressing force suppressing unit 60 as the external force along the first direction DR1, it is possible to effectively suppress the modification of the rear cover portion 304 due to the external force by the modification suppressing units 361 and 362.

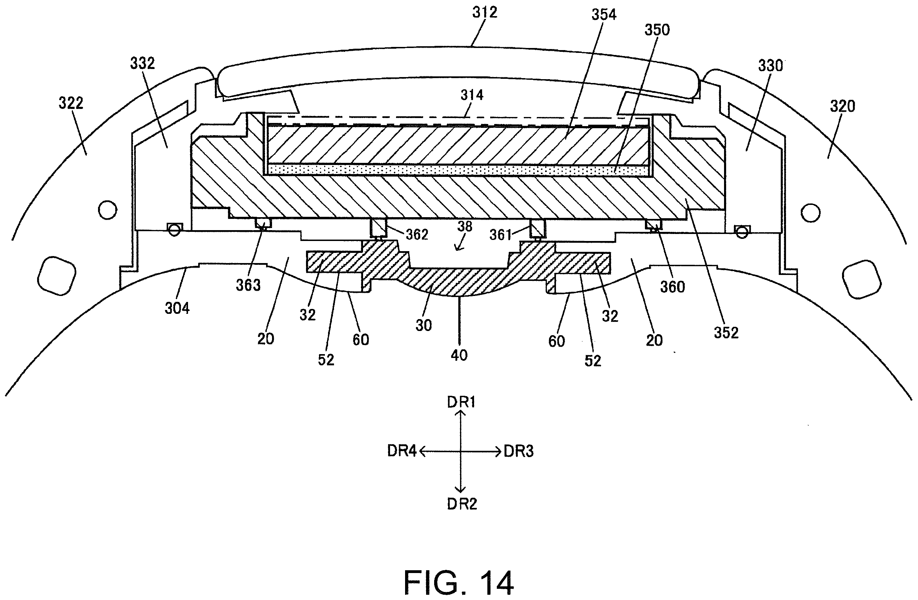

Furthermore, in Fig. 14, a sectional view illustrating another configuration example is illustrated in which the modification suppressing units 360, 361, 362, and 363 are disposed in the case portion 300. In Fig. 14, the shape of the circuit case 352 or the disposed state of the circuit substrate 350, the panel frame 354, and the display panel 314 is different from those in Fig. 13.

### 5. Configuration of Biological Information Detecting Device

In Fig. 15, a functional block diagram of a configuration example of the biological information detecting device of this embodiment is illustrated. In Fig. 15, the biological information detecting device includes the sensor unit 130, a motion sensor unit 170, a vibration generating unit 180, the processing unit 200, the storage unit 240, the communication unit 250, an antenna 252, and a notification unit 260. Furthermore, the configuration of the biological information detecting device of this embodiment is not limited to the configuration of Fig. 15, and various modifications are able to be performed in which a part of the constituents is omitted, a part of the constituents is substituted with other constituents, other constituents are added to the configuration, or the like.

The sensor unit 130 detects the biological information such as a pulse wave, and includes the light receiving unit 140 and the light emitting unit 150. A pulse wave sensor (a photoelectric sensor) is realized by the light receiving unit 140, the light emitting unit 150, and the like. The sensor unit 130 outputs a signal detected by the pulse wave sensor as a detection signal (a pulse wave detection signal).

The motion sensor unit 170 (a body motion detection unit) outputs the body motion detection signal which is a signal changed according to a body motion, on the basis of sensor information of various motion sensors. The motion sensor unit 170, for example, includes an acceleration sensor 172 as a motion sensor. Furthermore, the motion sensor unit 170 may include a pressure sensor, a gyro sensor, or the like as the motion sensor.

The processing unit 200, for example, performs various signal processings or control processings by using the storage unit 240 as a work region, and for example, is able to be realized by a processor such as CPU or a logic circuit such as ASIC. The processing unit 200 includes a signal processing unit 210, a beat information arithmetic unit 220, and a notification control unit 230.

The signal processing unit 210 performs various signal processings (filter processing or the like), and for example, performs signal processing according to a pulse wave detection signal from the sensor unit 130, a body motion detection signal from the motion sensor unit 170, or the like. For example, the signal processing unit 210 includes a body motion noise reducing unit 211. The body motion noise reducing unit 211 performs processing of reducing (eliminating) a body motion noise which is a noise caused by a body motion from the pulse wave detection signal on the basis of the body motion detection signal from the motion sensor unit 170. Specifically, for example, the body motion noise reducing unit 211 performs noise reduce processing using an adaptive filter or the like.

The beat information arithmetic unit 220 performs arithmetic processing with respect to beat information on the basis of the signal from the signal processing unit 210, and the like. The beat information, for example, is information such as a pulse rate. Specifically, the beat information arithmetic unit 220 performs frequency analytical processing such as FFT with respect to the pulse wave detection signal after being subjected to the noise reduce processing of the body motion noise reducing unit 211, obtains a spectrum, and performs processing in which a representative frequency in the obtained spectrum is set to a frequency of a heart rate. A value in which the obtained frequency is set to be 60 times becomes a pulse rate (a heart rate) which is generally used. Furthermore, the beat information is not limited to the pulse rate, and for example, may be various information items (for example, a frequency, a period, or the like of the heart rate) indicating the pulse rate. In addition, the beat information may be information indicating the state of the beat, and for example, a value indicating the amount of blood may be the beat information.

The notification control unit 230 controls the notification unit 260. The notification unit 260 (a notification device) notifies the user of various information items by the control of the notification control unit 230. For example, a light emitting unit for notification is able to be used as the notification unit 260. In this case, the notification control unit 230 controls a current flowing LED, and thus, controls lighting, blinking, or the like of the light emitting unit. Furthermore, the notification unit 260 may be a display unit of LCD, a buzzer, or the like.

In addition, the notification control unit 230 controls the vibration generating unit 180. The vibration generating unit 180 notifies the user of various information items by a vibration. The vibration generating unit 180, for example, is able to be realized by a vibration motor (a vibrator). The vibration motor, for example, generates a vibration by rotating an offset weight. Specifically, a weight offset to both ends of driving shaft (a rotor shaft) is attached to a motor, and thus, the motor itself swings. The vibration of the vibration generating unit 180 is controlled by the notification control unit 230. Furthermore, the vibration generating unit 180 is not limited to such a vibration motor, but various modifications are able to be performed. For example, the vibration generating unit 180 may be realized by a piezo element or the like.

For example, a notification of start-up at the time of turning on a power source, a notification of the first success of pulse wave detection, a warning when a state of not enabling a pulse wave to be detected is continue for a predetermined period of time, a notification when a fat combustion zone is moved, a warning when a battery voltage decreases, a notification of wake-up alarm, a notification of mails or phone calls from a terminal device such as a smart phone, and the like are able to be performed according to the vibration generated by the vibration generating unit 180. Furthermore, such information may be notified by the light emitting unit for notification, or may be notified by both of the vibration generating unit 180 and the light emitting unit.

The communication unit 250, for example, performs communication processing such as near field communication with respect to an external terminal device. Specifically, the communication unit 250 performs processing of receiving a signal from the antenna 252 and processing of transmitting a signal to the antenna 252. The function of the communication unit 250 is able to be realized by a processor for communication or a logic circuit such as ASIC.

In Fig. 16, a functional block diagram of another configuration example of the biological information detecting device of this embodiment is illustrated. The configuration of Fig. 16 is different from that of Fig. 15 in the configuration of the sensor unit 130 and the signal processing unit 210.

In Fig. 16, the sensor unit 130 includes a first light receiving unit 141 and a second light receiving unit 142, and the light emitting unit 150. Furthermore, the sensor unit 130 may include three or more light receiving units. In addition, here, an example is described in which the light emitting unit is shared by a plurality of light receiving units, but the number of light emitting units is not limited to one, and two or more light emitting units may be included.

The pulse wave sensor (the photoelectric sensor) is realized by the first light receiving unit 141 and the second light receiving unit 142, the light emitting unit 150, and the like. According to a case of Fig. 16, a first pulse wave sensor is realized by the first light receiving unit 141 and the light emitting unit 150, and a second pulse wave sensor is realized by the second light receiving unit 142 and the light emitting unit 150. The sensor unit 130 outputs signals detected by a plurality of pulse wave sensors as the detection signal (the pulse wave detection signal).

The signal processing unit 210 includes a body motion noise reducing unit 212 and a second body motion noise reducing unit 214. The body motion noise reducing unit 212 performs body motion noise reducing processing of reducing (eliminating) a body motion noise which is a noise caused by a body motion from a first detection signal of the first light receiving unit 141 on the basis of a second detection signal of the second light receiving unit 142 in the pulse wave detection signal. In addition, the second body motion noise reducing unit 214 performs second body motion noise reducing processing of reducing the body motion noise from the first detection signal on the basis of the body motion detection signal from the motion sensor unit 170. Specifically, a spectral subtraction may be used in the body motion noise reducing processing of the body motion noise reducing unit 212 , and an adaptive filter or the like may be used in the second body motion noise reducing processing of the second body motion noise reducing unit 214. Furthermore, in Fig. 16, a configuration is illustrated in which the second body motion noise reducing processing of the second body motion noise reducing unit 214 is performed after the body motion noise reducing processing of the body motion noise reducing unit 212 , but various modifications are able to be performed in which the processing order is reversed.

In a case of the configuration example of Fig. 16, for example, the first light receiving unit 141 is disposed between the light emitting unit 150 and the second light receiving unit 142. In a case where Fig. 10 is used as an example, the first light receiving unit 141 is disposed on the fourth direction DR4 side of the light emitting unit 152, and the second light receiving unit 142 is disposed on the fourth direction DR4 side of the first light receiving unit 141. Then, in a case where the signal processing unit 210 of the processing unit 200, for example, performs arithmetic processing with respect to the biological information of the test body on the basis of the first detection signal detected by the first light receiving unit 141, various processings such as noise reduce processing with respect to the first detection signal, or the like are performed on the basis of the second detection signal detected by the second light receiving unit 142.

In addition, a first convex portion corresponding to the first light receiving unit 141 and a second convex portion corresponding to the second light receiving unit 142 are disposed in the light transmitting portion 30. Here, the curvature of the first convex portion becomes larger than the curvature of the second convex portion. Then, in order to receive information for eliminating a noise signal, the second light receiving unit 142 acquires information of light incident through the second convex portion having a curvature different from that of the first convex portion on the first light receiving unit 141 side.

In addition, the first convex portion further protrudes to the test body side than the second convex portion. For example, the height of the first convex portion becomes higher than the height of the second convex portion. Specifically, the height of the first convex portion to the apex of a curved surface shape becomes higher than the height of the second convex portion to the apex of a curved surface shape. Here, the "height", for example, is a height from the substrate 160 of the sensor unit 130, and a height in the second direction DR2 of Fig. 10. In other words, in the second direction DR2 of Fig. 10, in a case where a height in a position or a region corresponding to the first light receiving unit 141 in the light transmitting portion 30 is set to h1, and a height in a position or a region corresponding to the second light receiving unit 142 in the light transmitting portion 30 is set to h2, h1 > h2 is set. Here, the definition of the position or the region corresponding to each light receiving unit is also variously considered. For example, the height h1 may be the height of the light transmitting portion 30 in a representative position of the first light receiving unit 141, and the height h2 may be the height of the light transmitting portion 30 in a representative position of the second light receiving unit 142. Here, for example, the center position of each light receiving unit, and the like may be used as the representative position. Alternatively, in a planar view seen from the test body side, in a case where a region including the first light receiving unit 141 and the light emitting unit 150 is set to a first region, and a region including the second light receiving unit 142 and the light emitting unit 150 is set to a second region, the height h1 may be the average height of the light transmitting portion 30 in the first region, and the height h2 may be the average height of the light transmitting portion 30 in the second region.

In a case where biological information such as pulse wave information is detected by using a photoelectric sensor (the sensor unit), a noise due to a body motion becomes a problem. For this reason, in order to detect the biological information with high accuracy, it is necessary to reduce a body motion noise by any method.

At the time of reducing the body motion noise, a component corresponding to a pulse signal in detection signals of the photoelectric sensor is maximally maintained, and a component corresponding to a body motion noise is reduced (in a narrow sense, eliminated). That is, in processing of reducing the body motion noise, it is necessary to grasp a signal component corresponding to the body motion noise.

From this viewpoint, in the configuration example of Fig. 15, the body motion noise is reduced by only using the motion sensor unit 170 such as an acceleration sensor. The motion sensor unit 170 is a sensor detecting the movement of the user (a wearer of the biological information detecting device), and thus, it is possible to acquire a signal corresponding to a body motion, that is, the signal corresponding to the body motion noise by using the motion sensor unit 170.

On the other hand, in the configuration example of Fig. 16, a method is adopted in which the second light receiving unit 142 different from the first light receiving unit 141 detecting a pulse signal is prepared, and acquires a signal including a large number of body motion noises.

That is, the body motion noise is included in the detection signal of the photoelectric sensor. By using such a viewpoint, in the second light receiving unit 142, the sensitivity of the pulse signal is set to be low, and the sensitivity of the body motion noise is set to be high, and thus, the detection signal mainly including the body motion noise is acquired.

In a case where the signal corresponding to the body motion noise is able to be detected in the second light receiving unit 142, a component corresponding to the detection signal of the second light receiving unit 142 is eliminated (reduced) from the detection signal of the first light receiving unit 141, and thus, the body motion noise is able to be reduced. At this time, the sensitivity of the pulse signal is low in the second light receiving unit 142, and thus, it is possible to avoid a pulse component included in the detection signal of the first light receiving unit 141 from being excessively reduced.

Here, in order to enable such processing to be performed, it is desirable that properties of the body motion noise included in the detection signal (for example, frequency properties) are coincident with each other or sufficiently close to each other in the first light receiving unit 141 and the second light receiving unit 142. That is, a high mutual relationship in the detection signals of two light receiving units is retained while providing a difference in detection properties such that the first light receiving unit 141 mainly detects the pulse signal, and the second light receiving unit 142 mainly detects the body motion noise. Specifically, wavelength ranges of light rays used in the configuration example of Fig. 16, for example, are identical to each other in the first light receiving unit 141 and the second light receiving unit 142. Furthermore, the light rays in the same wavelength range do not indicate that wavelengths at which intensity is maximized are completely identical to each other, but indicate that the wavelengths at which the intensity is maximized are included in a predetermined range (for example, a range of the same color). Light output from the light emitting unit 150, for example, is light in a wavelength range included in a range of greater than or equal to 470 nm and less than or equal to 610 nm. More specifically, the light output from the light emitting unit 150 is light in a wavelength range included in a range of greater than or equal to 520 nm and less than or equal to 570 nm. The light in this wavelength range is easily reflected on hemoglobin in a blood vessel, compared to other wavelengths.

Then, for example, a difference is provided in at least one of the height of the light transmitting portion 30 in the position or the region corresponding to each light receiving unit, and a distance between each light receiving unit and the light emitting unit, and thus, the first light receiving unit 141 is able to mainly detect the pulse signal, and the second light receiving unit 142 is able to mainly detect the body motion noise. Then, body motion noise reducing processing using the second detection signal of the second light receiving unit 142 is performed with respect to the first detection signal of the first light receiving unit 141, and thus, detection accuracy or detection sensitivity of biological information is able to be improved.

Furthermore, as described above, this embodiment has been described in detail, but a person skilled in the art will be easily understood that various modifications not deviating from the new matters and the effects of the present invention are able to be performed. Accordingly, all of the modification examples are included in the range of the present invention. For example, in the specification or the drawings, terms described at least once along with different terms having more comprehensive or synonymous meaning are able to be substituted with the different terms in any part of the specification or the drawings. In addition, the configuration and the operation of the biological information detecting device are not also limited to the description in this embodiment, but various modifications are able to be performed.

### Reference Signs List

2: skin
20: light blocking portion
22: convex portion
30: light transmitting portion
32: convex portion
40: convex portion (first convex portion)
42: groove portion
52: joining portion
60: pressing force suppressing unit (second convex portion)
70: light blocking member
81: opening portion
100: light blocking wall
120: detection window
130: sensor unit
140: light receiving unit
141, 142: first light receiving unit and second light receiving unit
150: light emitting unit
152: dome type lens
160: substrate
170: motion sensor unit
172: acceleration sensor
200: processing unit
210: signal processing unit
211: body motion noise reducing unit
212: first body motion noise reducing unit
214: second body motion noise reducing unit
220: beat information arithmetic unit
230: notification control unit
240: storage unit
250: communication unit
252: antenna
260: notification unit
300: case portion (main body portion)
302: top case
304: rear cover portion
310: display unit
312: windshield
320, 322: band portion
324: hook portion
326: pin
340: buckle portion
342: band insertion portion
344: rod portion
360, 361, 362, 363: modification suppressing unit (third convex portion)
400: wrist

## Claims

1. A biological information detecting device, comprising:
a sensor unit which detects biological information of a test body; and
a case portion in which the sensor unit is disposed,
wherein the case portion is provided with a light transmitting portion including a detection window which transmits light incident on the sensor unit, and a light blocking portion blocking light from being incident on an inner portion of the case portion, and
in a case in which a direction towards the sensor unit from the test body is set to a first direction at the time of mounting the biological information detecting device, a joining portion between the light transmitting portion and the light blocking portion protrudes or is dented along a direction intersecting with the first direction.

2. The biological information detecting device according to Claim 1,
wherein the light transmitting portion includes a first convex portion protruding in a direction opposite to the first direction.

3. The biological information detecting device according to Claim 1 or 2,
wherein a second convex portion disposed to surround the light transmitting portion is provided.

4. The biological information detecting device according to Claim 1,
wherein the light transmitting portion includes a first convex portion which imparts a pressing force by being in contact with the test body at the time of measuring the biological information, and
the light blocking portion includes a second convex portion which suppresses the pressing force imparted to the test body by the first convex portion.

5. The biological information detecting device according to Claim 4,
wherein the joining portion protrudes or is dented along the direction intersecting with the first direction on the first direction side of the second convex portion.

6. The biological information detecting device according to Claim 4 or 5,
wherein in a case in which a direction opposite to the first direction is set to a second direction, and a value obtained by subtracting a height of the second convex portion in the second direction from a height of the first convex portion in the second direction is set to Δh, Δh > 0 is satisfied.

7. The biological information detecting device according to any one of Claims 4 to 6,
wherein in a case in which a change amount of the pressing force of the first convex portion with respect to a load of a load mechanism generating the pressing force of the first convex portion is set to a pressing force change amount, the second convex portion suppresses the pressing force imparted to the test body by the first convex portion such that the pressing force change amount in a second load range in which the load of the load mechanism becomes greater than FL1 becomes smaller than the pressing force change amount in a first load range in which the load of the load mechanism becomes 0 to FL1.

8. The biological information detecting device according to any one of Claims 4 to 7,
wherein a groove portion is disposed between the first convex portion of the light transmitting portion and the second convex portion.

9. The biological information detecting device according to any one of Claims 1 to 8,
wherein the light transmitting portion and the light blocking portion are integrally formed by two-color molding or insert molding.

10. The biological information detecting device according to any one of Claims 1 to 9,
wherein the light transmitting portion is formed of a material including a first resin material, and
the light blocking portion is also formed of the material including the first resin material.

11. The biological information detecting device according to Claim 10,
wherein the light blocking portion is formed of a material in which glass is contained in the first resin material.

12. The biological information detecting device according to Claim 10 or 11,
wherein the first resin material is polycarbonate, an ABS resin, or an acrylic resin.

13. The biological information detecting device according to any one of Claims 1 to 12,
wherein the light blocking portion is joined to the light transmitting portion by being disposed to surround the detection window of the light transmitting portion, and
the joining portion between the light transmitting portion and the light blocking portion protrudes or is dented in a direction towards the light blocking portion from the detection window of the light transmitting portion.

14. The biological information detecting device according to any one of Claims 1 to 13,
wherein the light transmitting portion includes a convex portion protruding in a direction towards the light blocking portion from the detection window of the light transmitting portion, and
the light blocking portion includes a concave portion engaged with the convex portion of the light transmitting portion.

15. The biological information detecting device according to any one of Claims 1 to 13,
wherein the light blocking portion includes a convex portion protruding in a direction towards the detection window of the light transmitting portion from the light blocking portion, and
the light transmitting portion includes a concave portion engaged with the convex portion of the light blocking portion.

16. The biological information detecting device according to any one of Claims 1 to 15,
wherein the case portion is provided with a third convex portion which prevents at least one of the light transmitting portion and the light blocking portion from being modified in the first direction in the joining portion.

17. The biological information detecting device according to any one of Claims 1 to 15,
wherein a third convex portion is disposed between a circuit case supporting a circuit substrate and the light transmitting portion.

18. The biological information detecting device according to any one of Claims 1 to 17,
wherein the sensor unit includes a light emitting unit emitting light to the test body, and a light receiving unit receiving light from the test body.

19. The biological information detecting device according to any one of Claims 1 to 18,
wherein the biological information is pulse wave information.
